# EUROPEAN PATENT APPLICATION

(11) **EP 4 516 906 A1**
(43) Date of publication of application: **05.03.2025**
(21) Application number: 23796149.5
(22) Date of filing: 14.04.2023
(51) Int. Cl.: C12N 15/12, A01K 67/027, A61K 35/76, A61K 38/17, A61K 39/00, A61K 48/00, A61P 25/16, A61P 25/28, A61P 37/04, C07K 14/47, C07K 16/18, C12N 5/10, C12N 15/63, C12Q 1/02

(54) **VARIANT NEURODEGENERATIVE DISEASE-ASSOCIATED PROTEIN**

(30) Priority: 25.04.2022 JP 2022071735
(71) Applicant: Tokyo Metropolitan Institute of Medical Science, Tokyo 156-8506 (JP)
(72) Inventor: NONAKA Takashi, Tokyo 156-8506 (JP)
(74) Representative: J A Kemp LLP
(86) International application number: PCT/JP2023/015155
(87) International publication number: WO 2023/210405

(57) **Abstract**

A variant neurodegenerative disease-associated protein, which comprises an amino acid sequence comprising a deletion, substitution or addition of one or several amino acids present in the interaction region of two molecules of protofilaments (PF) in the amino acid sequence of a neurodegenerative disease-associated protein, and in which seed activity that functions as a nucleus of an aggregate of the neurodegenerative disease-associated protein is reduced.

## Description

### Technical Field

The present invention relates to a variant neurodegenerative disease-associated protein, a vaccine against neurodegenerative disease, and the like.

### Background Art

Immunotherapy is being vigorously developed as a novel therapeutic agent for dementia. Aducanumab (Patent Literature 1), approved in the United States in June 2021, is the world's first antibody drug that targets the accumulation of amyloid-β (Aβ) in Alzheimer's disease. This drug is a monoclonal antibody against Aβ and is administered to patients as antibody therapy (passive immunotherapy). In contrast, active immunotherapy, so-called vaccine therapy, in which a certain antigen is inoculated into a patient so that the patient is allowed to produce antibodies, is also under development (Immunization with amyloid-beta attenuates Alzheimer-disease-like pathology in the PDAPP mouse. Schenk D, et al. Nature; 400(6740): 173-177, 1999, Past, present and future of therapeutic strategies against amyloid-β peptides in Alzheimer's disease: a systematic review. Jeremic D, Jimenez-Diaz L, Navarro-Lopez JD. Ageing Res Rev. 2021 Dec; 72: 101496. doi: 10.1016/j.arr.2021.101496. Epub 2021 Oct 21).

However, it is said that only 0.1% of the antibodies in the blood are transferred into the brain. Thus, passive immunotherapy, which can introduce a large amount of antibody from the periphery, is considered to be more effective than vaccines, when it is considered as a therapeutic agent. However, since purified monoclonal antibodies are very expensive, the high cost of treatment is a major problem. For example, in the case of Aducanumab, the annual treatment cost per patient is said to be 6 million yen. Furthermore, in the case of antibody therapy for dementia, even if an antibody drug is administered to patients who have already developed the disease, it is said to be effective in removing aggregated proteins from the brain, but has little effect on improving cognitive functions.

In other words, nerve cell death has already occurred at the onset of the disease, and antibody therapy at this stage will not stop the progression of the disease. Therefore, antibody therapy for dementia is considered to be most effective when the antibody drug is administered to those who are in the "preclinical" stage in which there are no symptoms but pathological changes have begun, or in the "prodromal" stage in which symptoms are limited to the level of mild cognitive impairment. In such cases, it is expected that antibodies are administered even more than 10 years before the onset of the disease, and the treatment cost is expected to be high, especially in passive immunotherapy.

### Citation List

### Patent Literature

Patent Literature 1: US10,842,871 B2

### Non Patent Literature

Non Patent Literature 1: Disease-Modifying Therapies for Alzheimer's Disease: More Questions than Answers. Golde TE. Neurotherapeutics. 28:1-19. 2022.
Non Patent Literature 2: https://investors.biogen.com/news-releases/news-release-details/biogen-announces-reduced-price-aduhelmr-improve-access-patients

### Summary of Invention

### Technical Problem

Against the above-described background, it has been desired to develop a vaccine therapy against neurodegenerative diseases such as alpha-synucleinopathy that is a generic term for diseases in which alpha-synuclein accumulates in the brain (e.g. dementia with Lewy bodies (DLB), Parkinson's disease (PD), multiple system atrophy (MSA), etc.).

### Solution to Problem

The present inventor has conducted intensive studies directed towards achieving the aforementioned object, and as a result, the present inventor has succeeded in achieving the object, thereby completing the present invention.

Specifically, the present invention is as follows.
[1] A variant neurodegenerative disease-associated protein, which comprises an amino acid sequence comprising a deletion, substitution or addition of one or several amino acids in the amino acid sequence of the interaction region of two molecules of protofilaments (PF) in the amino acid sequence of a neurodegenerative disease-associated protein, and in which seed activity that functions as a nucleus of an aggregate of the neurodegenerative disease-associated protein is reduced to 70% or less of the seed activity of a wild-type neurodegenerative disease-associated protein.
[2] The variant neurodegenerative disease-associated protein according to the above [1], wherein the neurodegenerative disease-associated protein is any protein selected from alpha-synuclein, tau and amyloid beta.
[3] The variant neurodegenerative disease-associated protein according to the above [2], wherein the neurodegenerative disease-associated protein is alpha-synuclein, and one or several basic amino acids are deleted or substituted.
[4] The variant neurodegenerative disease-associated protein according to the above [3], wherein the basic amino acid is at least one selected from Lys43, Lys45 and His50.
[5] The variant neurodegenerative disease-associated protein according to the above [3], wherein the amino acid sequence of the alpha-synuclein is as set forth in SEQ ID No: 2.
[6] The variant neurodegenerative disease-associated protein according to the above [2], wherein the neurodegenerative disease-associated protein is tau, and one or several basic amino acids or an amino acid sequence comprising an amino acid sequence shown as PGGG are deleted or substituted.
[6-2] The variant neurodegenerative disease-associated protein according to the above [6], wherein the amino acid sequence comprising the amino acid sequence shown as PGGG is shown as KPGGGQ.
[7] The variant neurodegenerative disease-associated protein according to the above [6], wherein the basic amino acid is a basic amino acid comprised in any isoform selected from the 3R0N, 3R1N, 3R2N, 4R0N, 4R1N and 4R2N isoforms of tau, and it is at least one of His329, His330 and Lys331 in the 4R2N isoform, or at least one selected from basic amino acids corresponding to the His329, His330 and Lys331 in the isoforms other than the 4R2N.
[8] The variant neurodegenerative disease-associated protein according to the above [6], wherein the amino acid sequence of the tau is as set forth in SEQ ID No: 4, 6, 8, 10, 12 or 14.
[9] The variant neurodegenerative disease-associated protein according to the above [2], wherein the neurodegenerative disease-associated protein is amyloid beta, and one or several hydrophobic amino acids or basic amino acids are deleted or substituted.
[10] The variant neurodegenerative disease-associated protein according to the above [9], wherein the hydrophobic amino acid or the basic amino acid is at least one selected from Leu34, Val36 and Lys28.
[11] The variant neurodegenerative disease-associated protein according to the above [9], wherein the amino acid sequence of the amyloid beta is as set forth in SEQ ID No: 16.
[12] A nucleic acid encoding the variant neurodegenerative disease-associated protein according to any one of the above [1] to [11].
[13] A recombinant vector comprising the nucleic acid according to the above [12].
[14] An aggregate of the neurodegenerative disease-associated protein, in which the variant neurodegenerative disease-associated protein according to any one of the above [1] to [11] is aggregated.
[15] An antibody against the aggregate according to the above [14].
[16] A pharmaceutical composition against neurodegenerative disease, comprising the variant neurodegenerative disease-associated protein according to any one of the above [1] to [11].
[17] A pharmaceutical composition against neurodegenerative disease, comprising the nucleic acid according to the above [12].
[18] A pharmaceutical composition against neurodegenerative disease, comprising the recombinant vector according to the above [13].
[19] A vaccine against neurodegenerative disease, comprising the aggregate according to the above [14].
[20] A method for preventing or treating neurodegenerative disease, which is characterized in that it comprises administering the pharmaceutical composition according to the above [16] to a human.
[21] A method for preventing or treating neurodegenerative disease, which is characterized in that it comprises administering the pharmaceutical composition according to the above [17] to a human.
[22] A method for preventing or treating neurodegenerative disease, which is characterized in that it comprises administering the pharmaceutical composition according to the above [18] to a human.
[23] A method for preventing or treating neurodegenerative disease, which is characterized in that it comprises administering the vaccine according to the above [19] to a human.
[24] A variant neurodegenerative disease-associated protein: which comprises an amino acid sequence comprising a substitution of one or several hydrophobic amino acids or basic amino acids with basic amino acids in the amino acid sequence of the interaction region (PF interaction region) of two molecules of protofilaments (PF) in the amino acid sequence of a neurodegenerative disease-associated protein (provided that when a basic amino acid(s) are substituted, they are substituted with other basic amino acid(s) having a higher charge than the concerned basic amino acids), or an amino acid sequence comprising an addition of one or several basic amino acids to the PF interaction region; and which has seed activity of functioning as a nucleus of an aggregate of the neurodegenerative disease-associated protein that is 71% or more compared with the seed activity of a wild-type neurodegenerative disease-associated protein.
[25] The variant neurodegenerative disease-associated protein according to the above [24], wherein the neurodegenerative disease-associated protein is any protein selected from alpha-synuclein, tau and amyloid beta.
[26] The variant neurodegenerative disease-associated protein according to the above [25], wherein the neurodegenerative disease-associated protein is alpha-synuclein.
[27] The variant neurodegenerative disease-associated protein according to the above [26], wherein the one or several basic amino acids in the PF interaction region are at least one selected from Lys43, Lys45 and His50.
[28] The variant neurodegenerative disease-associated protein according to the above [26], wherein the amino acid sequence of the alpha-synuclein is as set forth in SEQ ID No: 2.
[29] The variant neurodegenerative disease-associated protein according to the above [25], wherein the neurodegenerative disease-associated protein is tau.
[30] The variant neurodegenerative disease-associated protein according to the above [29], wherein the one or several basic amino acids in the PF interaction region are basic amino acids comprised in any isoform selected from the 3R0N, 3R1N, 3R2N, 4R0N, 4R1N and 4R2N isoforms of tau, and the basic amino acid(s) are at least one of His329, His330 and Lys331 in the 4R2N isoform, or at least one selected from basic amino acids corresponding to the His329, His330 and Lys331 in the isoforms other than the 4R2N.
[31] The variant neurodegenerative disease-associated protein according to the above [29], wherein the amino acid sequence of the tau is as set forth in SEQ ID No: 4, 6, 8, 10, 12 or 14.
[32] The variant neurodegenerative disease-associated protein according to the above [25], wherein the neurodegenerative disease-associated protein is amyloid beta.
[33] The variant neurodegenerative disease-associated protein according to the above [32], wherein the one or several hydrophobic amino acids or basic amino acids in the PF interaction region are at least one selected from Leu34, Val36 and Lys28.
[34] The variant neurodegenerative disease-associated protein according to the above [32], wherein the amino acid sequence of the amyloid beta is as set forth in SEQ ID No: 16.
[35] An aggregate of the neurodegenerative disease-associated protein, in which the variant neurodegenerative disease-associated protein according to any one of the above [24] to [34] is aggregated.
[36] A cell or a non-human animal, into which the aggregate according to the above [35] is introduced.
[37] A cell model or a non-human animal model of variant neurodegenerative disease, comprising the cell or non-human animal according to the above [36].
[38] A nucleic acid encoding the variant neurodegenerative disease-associated protein according to any one of the above [24] to [34].
[39] A recombinant vector comprising the nucleic acid according to the above [38].
[40] A transformed cell or a transformed non-human animal, comprising the recombinant vector according to the above [39].
[41] A cell model or a non-human animal model of neurodegenerative disease, comprising the transformed cell or the transformed non-human animal according to the above [40].
[42] A method of screening for a therapeutic agent for neurodegenerative disease, which is characterized in that it comprises contacting or administering a candidate substance to be tested to the cell model or the non-human animal model according to the above [37] or the cell model or the non-human animal model according to the above [41].
[43] A kit of screening for a therapeutic agent for neurodegenerative disease, comprising at least one selected from the group consisting of the aggregate according to the above [35], the nucleic acid according to the above [38], the recombinant vector according to the above [39], the transformed cell or the transformed non-human animal according to the above [40], and the transformed cell or the transformed non-human animal according to the above [41].

### Advantageous Effects of Invention

According to the present invention, provided is a variant neurodegenerative disease-associated protein with reduced seed activity that functions as a nucleus of an aggregate of a neurodegenerative disease-associated protein. An aggregate of the variant protein with reduced seed activity is useful as a vaccine against neurodegenerative disease. In addition, the variant protein with reduced seed activity and a nucleic acid encoding the variant protein are useful as biopharmaceuticals and for gene therapy against neurodegenerative diseases.

Moreover, according to the present invention, provided is a variant protein having seed activity that functions as a nucleus of an aggregate of a neurodegenerative disease-associated protein. A cell or a non-human animal into which an aggregate of the protein is introduced, or a cell or a non-human animal in which the protein is expressed, are useful as a cell model or an animal model of neurodegenerative disease.

### Brief Description of Drawings

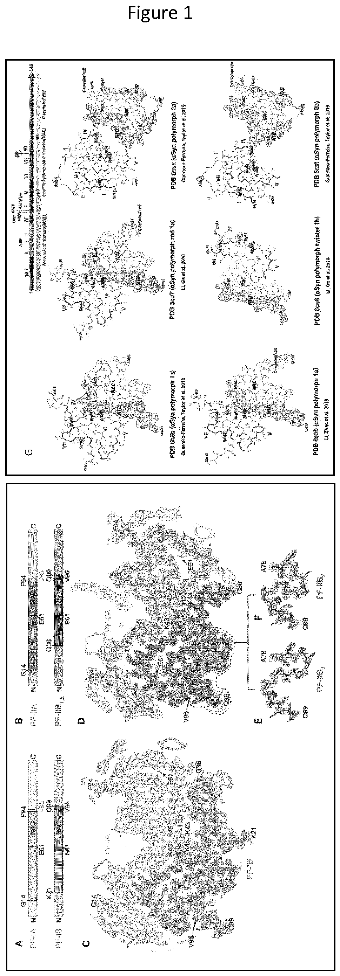

[Figure 1] Figure 1 is a view showing the three-dimensional structure of an alpha-synuclein (aS) aggregate according to cryo-electron microscopic analysis. Left panel (A-F): The structures of two aS aggregates prepared from the brains of multiple system atrophy (MSA) patients have been revealed, and each has a central core structure in which two molecules of aS protofilaments (amyloid fibril precursors: PFs) interact with each other. The first (A and C) is a structure consisting of two PFs, Gly14-Phe94 and Lys21-Gln99. The second structure (B-F) is a structure consisting of two PFs, Gly14-Phe94 and Gly36-Gln99, but some PFs of Gly36-Gln99 have a slightly different conformation (E and F). Right panel (G): Structures of recombinant aS aggregates. So far, several types of structures have been clarified. Basically, all of them have a central core structure consisting of two PF molecules interacting each other.

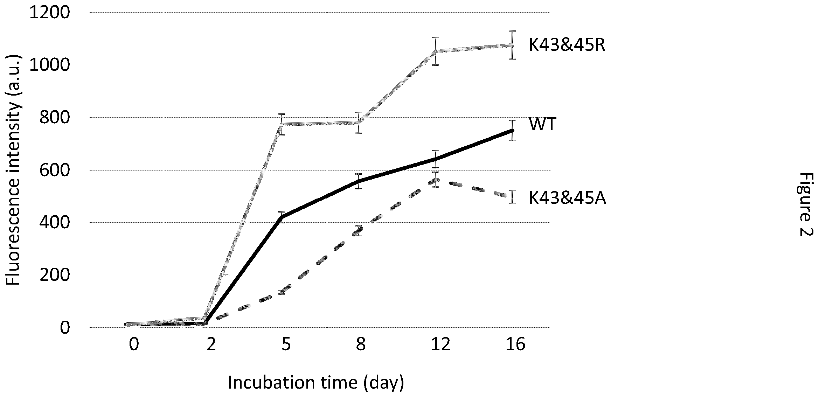

[Figure 2] Figure 2 is a view showing the aggregate formation of a recombinant alpha-synuclein monomer.
   Wild-type or variant aS monomer (1 mg/mL) was shaken at 37°C for aggregation. On 0, 2, 5, 8, 12 and 16 days after initiation of the shaking, samples were aliquoted and mixed with 20 mM Hepes (pH 7.5) containing 4 µM ThT, and the mixture was then incubated at 37°C for 30 minutes. The fluorescence intensity of the samples after the reaction was measured at an excitation wavelength of 442 nm and at a fluorescence wavelength of 485 nm. a.u.: arbitrary unit. Wild-type (WT) monomer: solid black line; K43&45A monomer: gray dotted line; and K43&45R monomer: gray solid line.

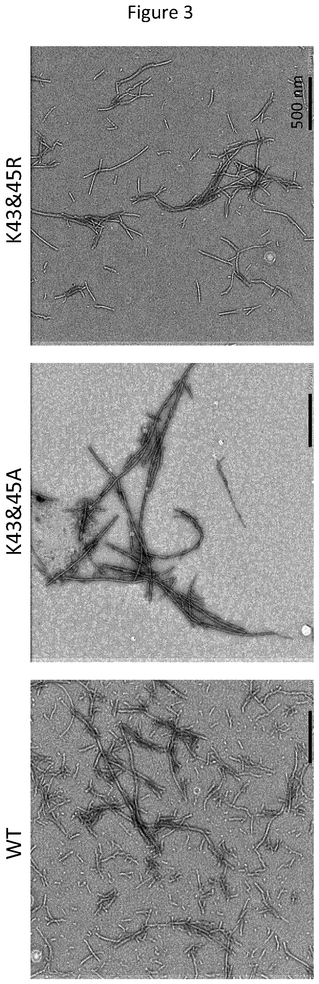

[Figure 3] Figure 3 shows electron microscopic images of alpha-synuclein aggregates. Each aggregate (0.05 mg/mL) was added to a collodion mesh and reacted with a 2% sodium phosphotungstate solution for negative staining. The stained mesh was subjected to JEM-1400 electron microscopy to observe the fiber structure of each aggregate. Fibrous structures were observed in both wild-type (WT) and variant aS aggregates.

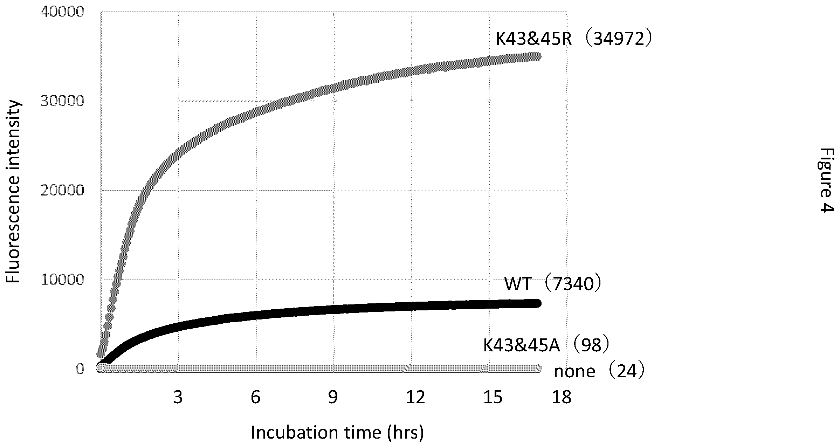

[Figure 4] Figure 4 is a view showing the seed activity (1) of aS aggregates *in vitro. A* wild-type aS monomer solution (1 mg/mL, 100 µL) was mixed with an aggregate (wild-type WT, K43&45A, and K43&45R: each 1 µg) , and the mixture was then incubated at 37°C. The fluorescence intensity of the reaction solution was continuously measured at an excitation wavelength of 442 nm and at a fluorescence wavelength of 485 nm. Incubation of the wild-type monomer alone at 37°C does not increase the fluorescence intensity of ThT (no aggregation), but addition of the wild-type or K43&45R aggregate thereto causes seed-dependent aggregation. However, the wild-type monomer did not aggregate at all when the K43&45A aggregate was added. The numerical values in the parentheses indicate the fluorescence values at the final measurement time point.

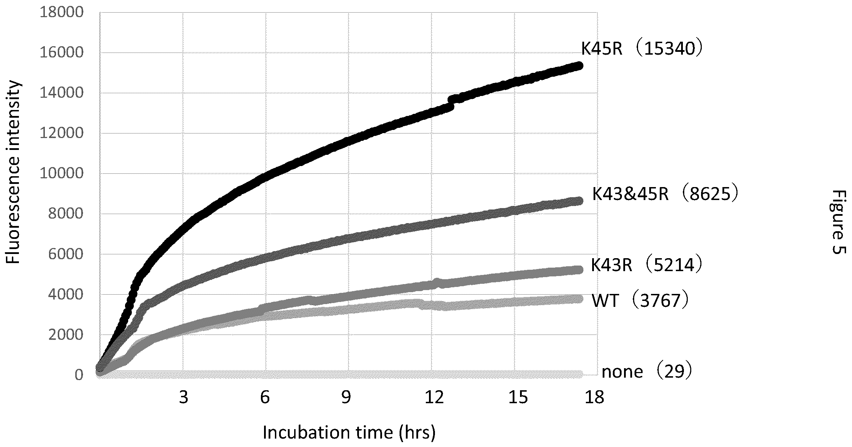

[Figure 5] Figure 5 is a view showing the seed activity (2) of aS aggregates *in vitro. A* wild-type aS monomer solution (1 mg/mL, 100 µL) was mixed with an aggregate (wild-type WT, K43R, K45R, and K43&45R: each 1 µg) , and the mixture was then incubated at 37°C. The fluorescence intensity of the reaction solution was continuously measured at an excitation wavelength of 442 nm and at a fluorescence wavelength of 485 nm. Incubation of the wild-type monomer alone (none) at 37°C does not increase the fluorescence intensity of ThT (no aggregation), but addition of the wild-type or other aggregates thereto causes seed-dependent aggregation. The numerical values in the parentheses indicate the fluorescence values at the final measurement time point. The seed activity of each aggregate is as follows. The variant aggregates all had stronger seeding effects than the wild-type aggregate. K45R aggregate> K43&45R aggregate > K43R aggregate > WT aggregate

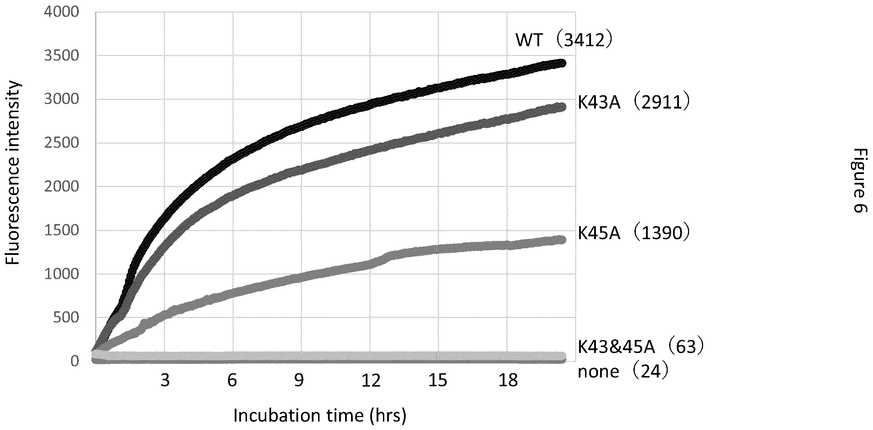

[Figure 6] Figure 6 is a view showing the seed activity (3) of aS aggregates *in vitro. A* wild-type aS monomer solution (1 mg/mL, 100 µL) was mixed with an aggregate (wild-type WT, K43A, K45A, and K43&45A: each 1 µg) , and the mixture was then incubated at 37°C. The fluorescence intensity of the reaction solution was continuously measured at an excitation wavelength of 442 nm and at a fluorescence wavelength of 485 nm. Incubation of the wild-type monomer alone (none) at 37°C does not increase the fluorescence intensity of ThT (no aggregation), but addition of the wild-type or other aggregates thereto causes seed-dependent aggregation. The numerical values in the parentheses indicate the fluorescence values at the final measurement time point. The seed activity of each aggregate is as follows: WT aggregate > K43A aggregate > K45A aggregate > K43&45A aggregate.

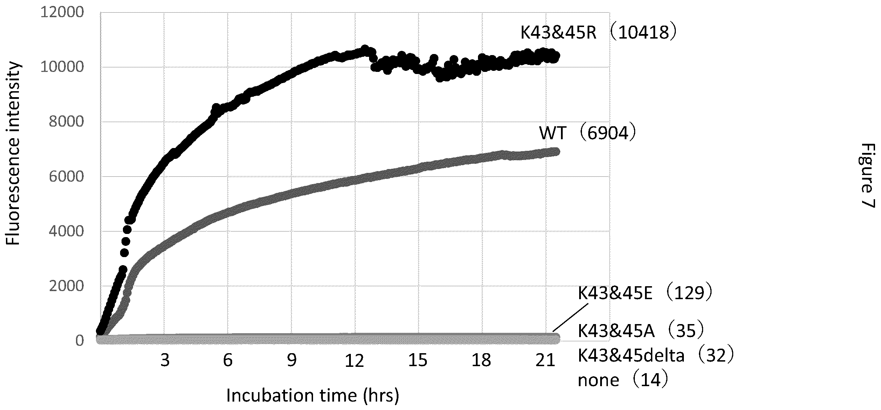

[Figure 7] Figure 7 is a view showing the seed activity (4) of aS aggregates *in vitro. A* wild-type aS monomer solution (1 mg/mL, 100 µL) was mixed with an aggregate (wild-type WT, K43&45A, K43&45E, K43&45R, and K43&45 delta: each 1 µg) , and the mixture was then incubated at 37°C. The fluorescence intensity of the reaction solution was continuously measured at an excitation wavelength of 442 nm and at a fluorescence wavelength of 485 nm. Incubation of the wild-type monomer alone (none) at 37°C does not increase the fluorescence intensity of ThT (no aggregation), but addition of the wild-type or K43&45R aggregate thereto causes seed-dependent aggregation. The numerical values in the parentheses indicate the fluorescence values at the final measurement time point. The seed activity of each aggregate is as follows: K43&45R aggregate > WT aggregate >> K43&45E aggregate > K43&45A aggregate > K43&45 delta aggregate.

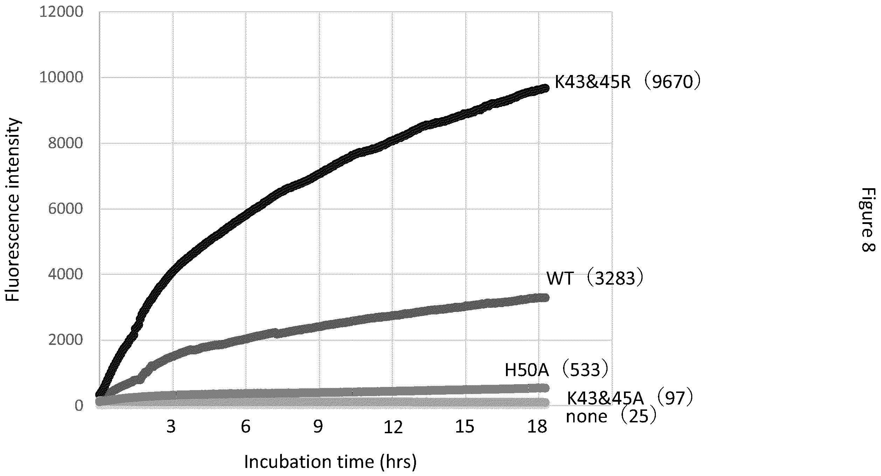

[Figure 8] Figure 8 is a view showing the seed activity (5) of aS aggregates *in vitro. A* wild-type aS monomer solution (1 mg/mL, 100 µL) was mixed with an aggregate (wild-type WT, K43&45A, K43&45R, and H50A: each 1 µg) , and the mixture was then incubated at 37°C. The fluorescence intensity of the reaction solution was continuously measured at an excitation wavelength of 442 nm and at a fluorescence wavelength of 485 nm. Incubation of the wild-type monomer alone (none) at 37°C does not increase the fluorescence intensity of ThT (no aggregation), but addition of the wild-type or other aggregates thereto causes seed-dependent aggregation. The numerical values in the parentheses indicate the fluorescence values at the final measurement time point. The seed activity of each aggregate is as follows: K43&45R aggregate > WT aggregate > H50A aggregate > K43&45A aggregate.

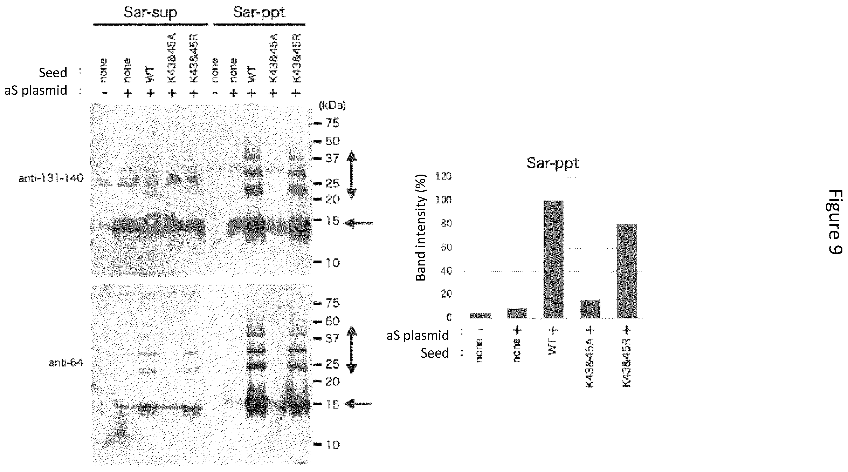

[Figure 9] Figure 9 is a view showing the seed activity of aS aggregates using cultured cells. Various aggregates (wild-type WT, K43&45A, and K43&45R aggregates: each 0.24 µg) were added as seeds to SH-SY5Y cells in which a wild-type aS plasmid was allowed to transiently express. The cells were recovered, were then homogenized with sarkosyl, one type of detergent, and were then centrifuged to obtain sarkosyl-soluble (Sar-sup) and - insoluble (Sar-ppt) fractions. These were subjected to immunoblotting using anti-131-140 (an antibody recognizing the C-terminus of aS) and anti-64 (an antibody recognizing phosphorylated aS). The amount of bands in the Sar-ppt fractions with the anti-64 antibody was quantified using Image J, and the amount of aS that accumulated in the cells was shown. Arrow: aS; and double-headed arrow: ubiquitinated aS or aS multimer: Among the three bands, the one around 25k is considered to be aS bound with one ubiquitin, the one around 30k is considered to be an aS dimer, and the one around 45k is considered to be aS bound with two ubiquitins, or an aS trimer.

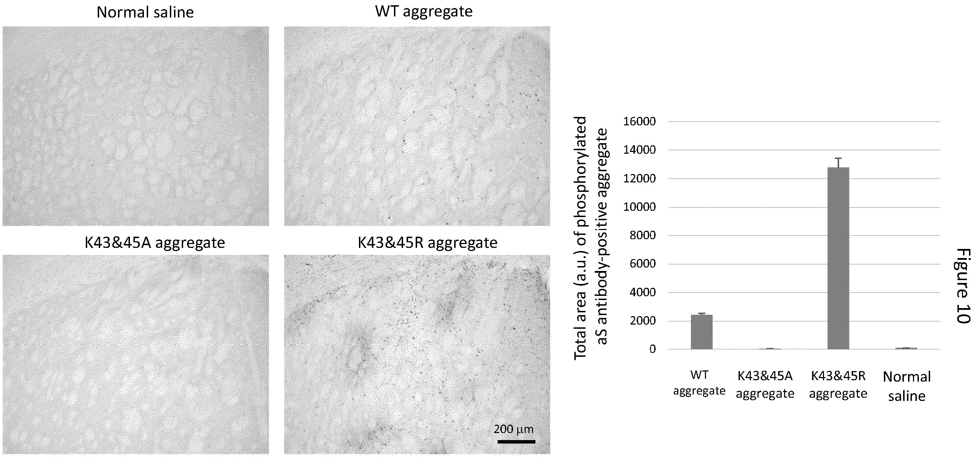

[Figure 10] Figure 10 is a view showing the prion-like activity of aS aggregates in mouse brain. Each aggregate (10 µg) was injected into the striatum of mouse brain, and the brain was excised 3 months later. The excised brain was immobilized and was stained with a phosphorylated aS-specific antibody (anti-pS129). In the brain (near the right cerebral cortex) into which a wild-type (WT) aggregate or a K43&45R aggregate was injected, many phosphorylated aS antibody-positive aggregates (round structures in the figure) are observed, whereas in the brain into which a normal saline or a K43&45A aggregate was injected, almost no phosphorylated aS aggregates were observed. a.u.: arbitrary unit. Scale bar: 200 µm.

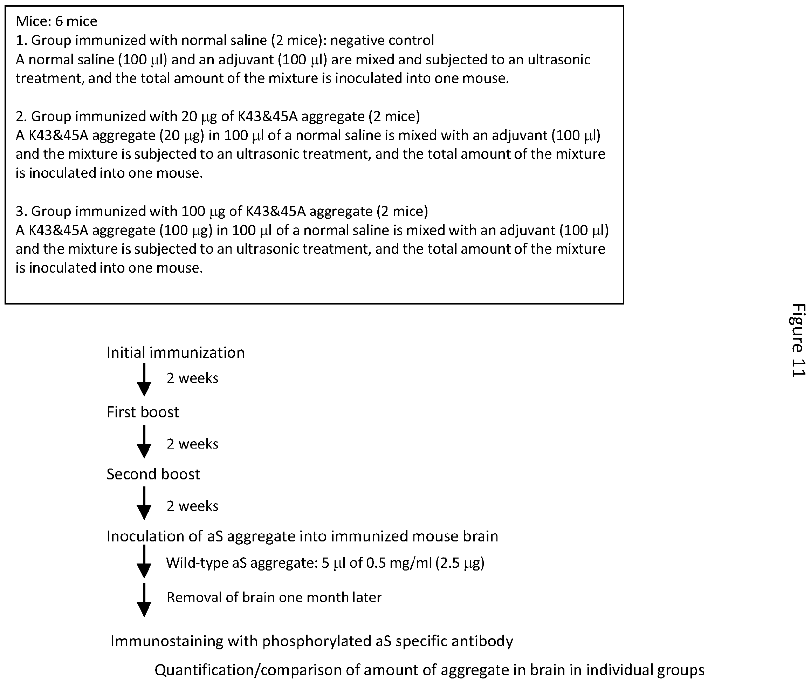

[Figure 11] Figure 11 is a view showing an outline of a vaccination test.

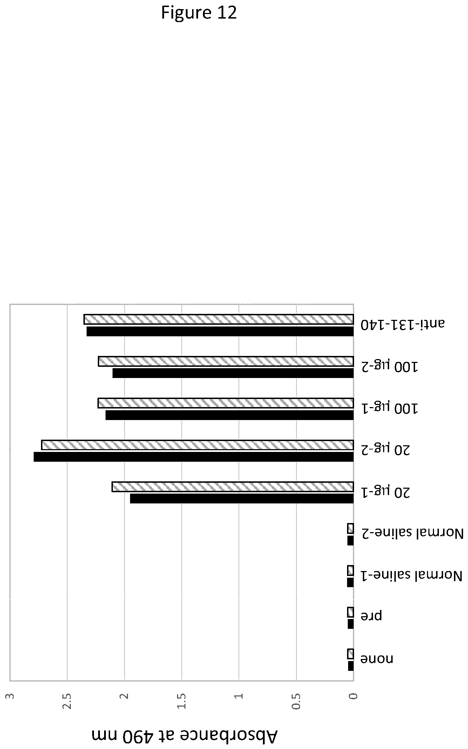

[Figure 12] Figure 12 is a view showing the results obtained by measuring the serum from vaccine-administered mice according to ELISA. Blood was collected from mice (2 mice each) immunized with a normal saline (saline), 20 µg of K43&45A aggregate, and 100 µg of K43&45A aggregate for a total of 3 times, and the serum fraction was obtained. These serum fractions were each added to a 96-well plate adsorbed with a mouse wild-type aS aggregate or a K43&45A aggregate, and the measurement was then performed according to an ELISA method. The reactivity to the mouse wild-type aS aggregate (absorbance at 490 nm) is shown in a black bar, and the reactivity to the K43&45A aggregate is shown in a shaded bar. An antibody recognizing the C-terminus of aS (anti-131-140) was used as a positive control. none: no serum; pre: mouse serum before vaccine administration; Normal saline-1, -2: groups inoculated with a normal saline; 20 µg-1, -2: groups inoculated with 20 µg of K43&45A aggregate; and 100 µg-1, -2: groups inoculated with 100 µg of K43&45A aggregate.

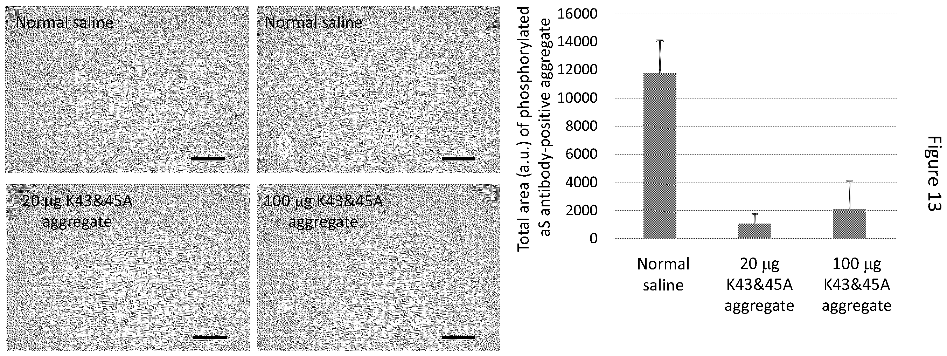

[Figure 13] Figure 13 is a view showing suppression (1) of aS aggregate formation in the mouse brain by vaccine administration. Mice immunized with a normal saline, 20 µg of K43&45A aggregate, and 100 µg of K43&45A aggregate were inoculated with aS fibers (2.5 µg) as seeds in the brain thereof. One month later, the brains were excised, and aS aggregate appearing in the mouse brains were then stained using phosphorylated aS antibody (anti-pS129 antibody) (image taken near the right cerebral cortex). The area values of anti-pS 129-positive aggregates were calculated from the stained images, using an all-in-one microscope (KEYENCE), and the values were then compared (lower graph). a.u.: arbitrary unit. Scale bar: 200 µm.

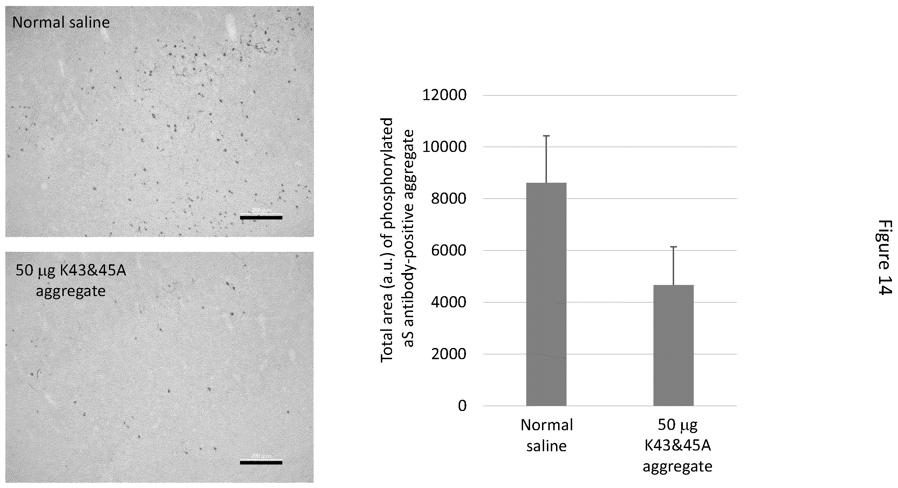

[Figure 14] Figure 14 is a view showing suppression (2) of aS aggregate formation in the mouse brain by vaccine administration. Mice immunized with a normal saline or 50 µg of K43&45A aggregate were inoculated with insoluble aS (2.5 µL) prepared from MSA patient brains as a seed in the brain thereof. One month later, the brains were excised, and aS aggregate appearing in the mouse brains were then stained using phosphorylated aS antibody (anti-pS129 antibody) (image taken near the right cerebral cortex). The area values of anti-pS 129-positive aggregates were calculated from the stained images, using an all-in-one microscope (KEYENCE), and the values were then compared (lower graph). a.u.: arbitrary unit. Scale bar: 200 µm.

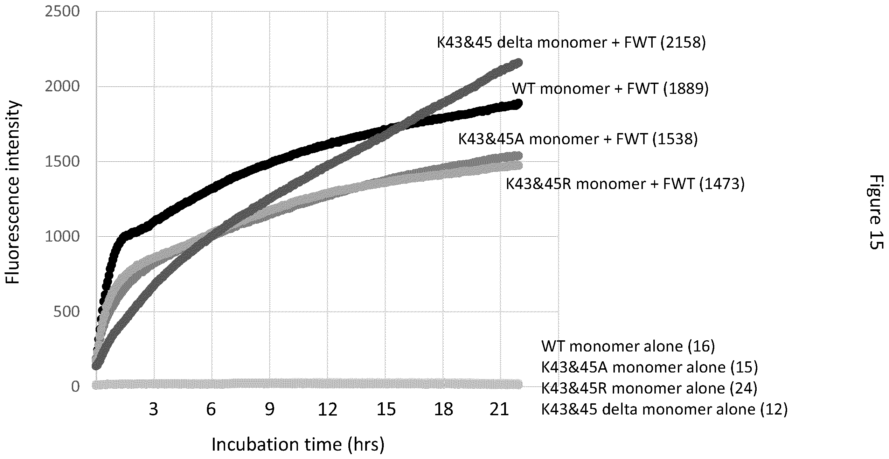

[Figure 15] Figure 15 is a view showing seed-dependent aggregation of aS monomer *in vitro.* Wild-type aS and variant aS monomer solutions (1 mg/mL, 100 µL) were mixed with wild-type aS aggregates (FWT: 1 mg each) by shaking, and each obtained mixture was then incubated at 37°C. The fluorescence intensities of the reaction solutions were continuously measured at an excitation wavelength of 442 nm and at a fluorescence wavelength of 485 nm. Incubation of each monomer alone at 37°C does not increase the fluorescence intensity of ThT (no aggregation), but when the wild-type aggregate (FWT) is added thereto, each monomer aggregates in a seed-dependent manner. The numerical values in the parentheses indicate the fluorescence values at the final measurement time point.

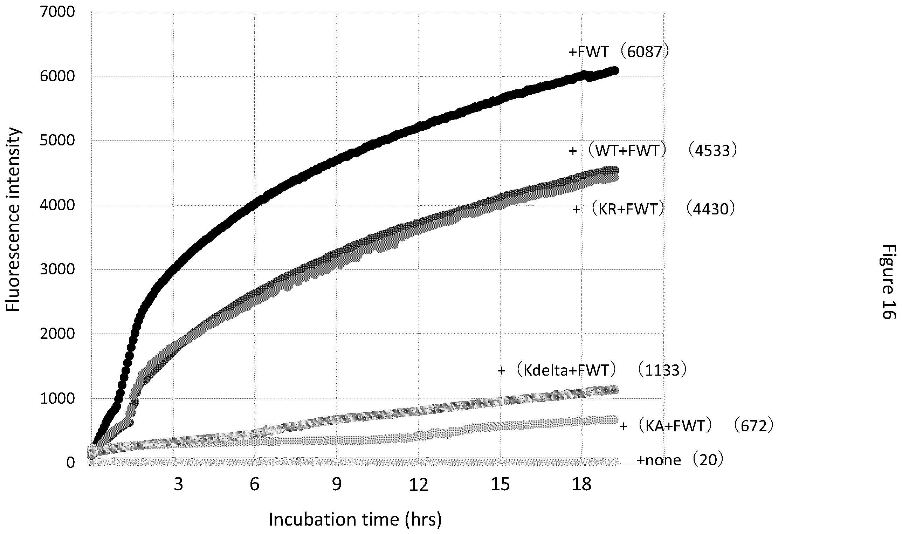

[Figure 16] Figure 16 is a view showing the seeding effects of various aS aggregates aggregated by addition of aS aggregate (FWT) by shaking. The wild-type aS monomer solution (1 mg/mL, 100 µL) was mixed with each seed-dependent aggregate (WT + FWT, KR + FWT, K delta + FWT, and KA + FWT: 1 µg each), and the obtained mixture was then incubated at 37°C. The fluorescence intensities of the reaction solutions were measured at 37°C. Fluorescence intensities of the reaction solutions were continuously measured at an excitation wavelength of 442 nm and at a fluorescence wavelength of 485 nm. The numerical values in the parentheses indicate the fluorescence value at the final measurement time point. WT: wild-type; KR: K43&45R; K delta: K43&45 delta; and KA: K43&45A.

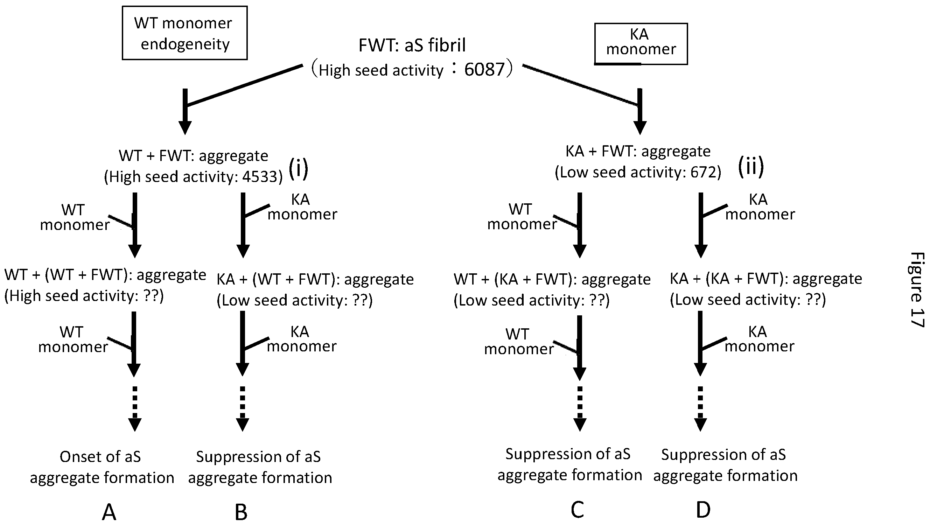

[Figure 17] Figure 17 shows a conceptual view of aS aggregate suppression by K43&45A monomer expression. WT: wild-type aS; KA: K43&45A; and FWT: WT aggregate obtained by shaking.

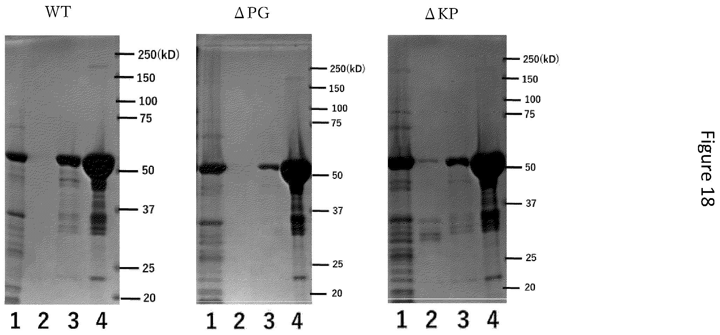

[Figure 18] Figure 18 is a view showing the results obtained by purifying recombinant tau monomers. The sample of a recombinant tau monomer at each purification stage was partially collected, and was then analyzed by SDS-PAGE (CBB staining). 1: heat-treated fraction; 2: 0.1 M NaCl wash fraction; 3: 0.35 M NaCl elution fraction; and 4: final product after dialysis (recombinant tau monomer).

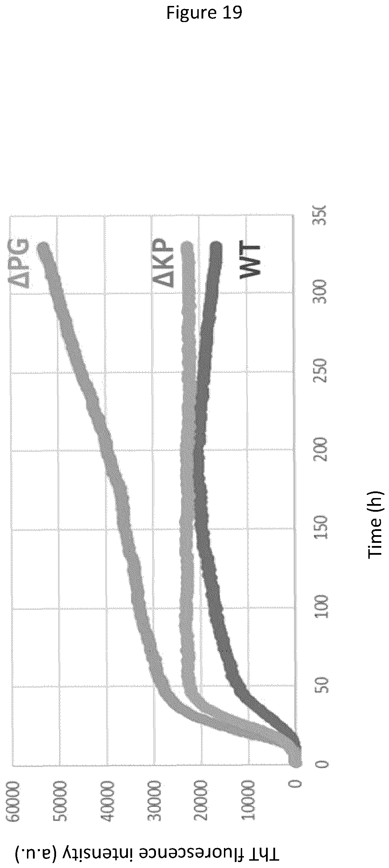

[Figure 19] Figure 19 is a view showing aggregation formation of recombinant tau monomers. Dextran sulfate and ThT were added to wild-type (WT) and variant monomers (ΔPG and ΔKP, 3 mg/ml each), and the fluorescence intensity of ThT was measured over time, using a FLUOstar Omega plate reader, at an excitation wavelength of 450 nm and at a fluorescence wavelength of 480 nm, while shaking at 37°C.

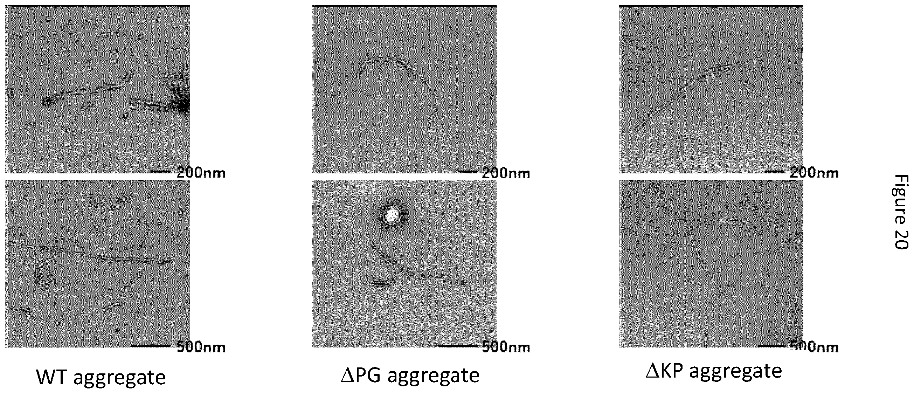

[Figure 20] Figure 20 shows observation images of individual aggregates according to electron microscopy. Wild-type (WT) and variant tau (ΔPG and ΔKP) aggregates (0.2 mg/ml, 2 µl) were placed on a mesh, and 2% phosphotungstic acid was then added thereto for negative staining, followed by observation according to electron microscopy.

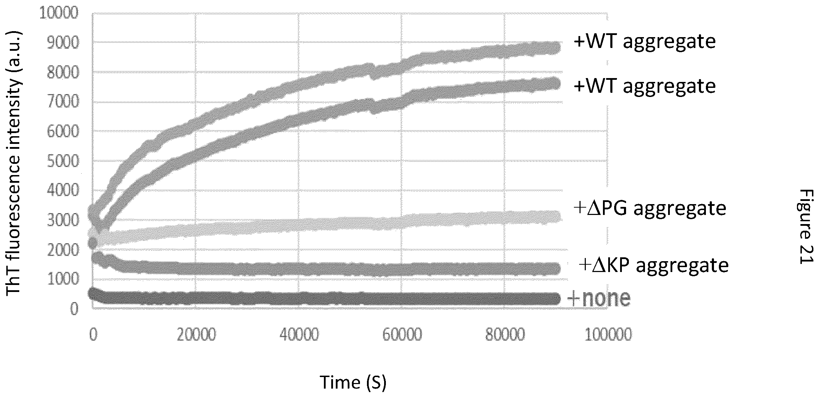

[Figure 21] Figure 21 is a view showing the seed activity of wild-type and variant aggregates *in vitro.* Each aggregate (1 mg/ml) and ThT were added to a wild-type tau monomer (1 mg/ml), and the obtained mixture was then incubated at 37°C using an infinite M200 PRO/infinite M NANO + plate reader (TECAN). The fluorescence intensity of ThT in these samples was measured over time at an excitation wavelength of 442 nm and at a fluorescence wavelength of 485 nm.

### Description of Embodiments

### 1. Outline

The present invention relates to a variant neurodegenerative disease-associated protein, which comprises an amino acid sequence comprising a deletion, substitution or addition of one or several amino acids in the amino acid sequence of the interaction region of two molecules of protofilaments (PF) in the amino acid sequence of a neurodegenerative disease-associated protein, and in which seed activity that functions as a nucleus of an aggregate of the neurodegenerative disease-associated protein is reduced.

In addition, in another aspect, the present invention relates to a variant neurodegenerative disease-associated protein: which comprises an amino acid sequence comprising a substitution of one or several hydrophobic amino acids or basic amino acids with basic amino acids in the amino acid sequence of the interaction region of two PF molecules in the amino acid sequence of a neurodegenerative disease-associated protein (provided that when a basic amino acid(s) are substituted, they are substituted with other basic amino acid(s) having a higher charge than the concerned basic amino acids), or an amino acid sequence comprising an addition of one or several basic amino acids to the PF interaction region; and which has seed activity of functioning as a nucleus of an aggregate of the neurodegenerative disease-associated protein.

In recent years, immunotherapy (vaccine/antibody therapy) has attracted attention as a fundamental therapeutic agent for various types of dementia. In the present invention, the present inventor has focused on a more inexpensive vaccine therapy and has aimed to develop a novel vaccine therapy against neurodegenerative diseases such as alpha-synucleinopathy. Based on the three-dimensional structure of an aggregate of neurodegenerative disease-associated protein accumulated in the brain of a patient or a recombinant aggregate, which is obtained by cryo-electron microscopy, the present inventor has developed a novel variant aggregate with almost no prion-like activity. Using this novel variant aggregate without prion-like activity, the present inventor has aimed to develop a highly safe and effective vaccine therapy. Moreover, the inventor has expressed a protein variant found by the present invention in the brain using adeno-associated virus (AAV), so that the inventor has attempted to develop a novel therapeutic method of suppressing accumulation of the neurodegenerative disease-associated protein in the brain.

The neurodegenerative disease-associated protein includes proteins such as alpha-synuclein (also referred to as "αS"), a tau protein (also simply referred to as "tau"), and amyloid beta (also referred to as "Aβ"). In these proteins, two molecules of protofilaments (PFs) are present. The present inventor has focused on the region in which these two molecules of PF interact with each other, and has produced variant proteins by deleting or substituting one or several amino acids present in this region, or by adding one or several amino acids to the region. Thereafter, the inventor has measured the seed activity thereof.

The term "seed activity" means the activity of causing molecular reactions that cause soluble proteins to polymerize to form insoluble aggregates, and the seed activity includes both the activity that causes protein monomers to aggregate to form aggregates and the activity by which the aggregates that have once aggregated serve as nuclei or seeds upon the aggregation of monomers, so as to form an aggregate.

The neurodegenerative disease-associated protein aggregates when it is shaken in the state of a monomer, but it does not aggregate if it is left at rest without shaking. When a small amount of previously prepared aggregate is added to a monomer solution that has been left at rest, the monomers aggregate (seed activity). However, by mutating some amino acids of the neurodegenerative disease-associated protein, an aggregate with reduced seed activity could be obtained.

As a result, it has been confirmed that a variant protein aggregate with reduced seed activity or a protein aggregate originally not having seed activity can function as a vaccine without promoting further aggregation of the monomers. The present invention has been completed based on these findings.

### 2. Variant neurodegenerative disease-associated protein with reduced seed activity

### 2.1. Mutation of amino acids and aggregates

The variant neurodegenerative disease-associated protein of the present invention is a protein comprising an amino acid sequence comprising a deletion, substitution or addition of one or several amino acids in the amino acid sequence of the interaction region of two molecules of protofilaments (PF) in the amino acid sequence of a neurodegenerative disease-associated protein, in which seed activity that functions as a nucleus of an aggregate of the neurodegenerative disease-associated protein is reduced. In the present section, the variant neurodegenerative disease-associated protein with reduced seed activity is also referred to as a "seed activity-reduced variant protein" or a "seed activity-reduced variant." In addition, an aggregate of the variant neurodegenerative disease-associated protein with reduced seed activity is also referred to as a "seed activity-reduced aggregate."

Herein, the "reduction" in the activity means that the seed activity functioning as a nucleus of an aggregate of the protein is reduced to 70% or less compared with the seed activity of a wild-type neurodegenerative disease-associated protein. Among others, the reduction in the activity to less than 10%, i.e., substantial or complete loss of the activity, is considered as "loss" of the activity. The loss of the activity also includes the complete loss of the activity (0%).

The activity can be measured, for example, by a fibrosis measurement test using thioflavin. In this case, the thioflavin value of a variant neurodegenerative disease-associated protein to be tested and the thioflavin value after addition of a wild-type seed are measured, and when the thioflavin values of both endpoints are reduced to 70% or less, it can be determined that the activity has been reduced.

Hereinafter, in the present description, unless otherwise stated, reducing of a seed activity functioning as a nucleus of a protein aggregate to 70% or less compared with the seed activity of a wild-type neurodegenerative disease-associated protein is simply referred to as "to reduce," and the variant protein is referred to as a "seed activity-reduced variant protein" or a "reduced variant protein."

The term "aggregate" refers to a deposited or accumulated insoluble protein structure that appears inside or outside of neurons and glial cells in the brain of patients with neurodegenerative diseases. The "aggregate" is a protein aggregate of neurodegenerative disease-associated proteins (monomers), including fibers as well as oligomers that are composed of several polymerized molecules. A fibrous protein aggregate is thought to be one of the pathological characteristics of many neurodegenerative diseases, and the formation process thereof is considered to be closely related to the onset of the disease. The pathological image of this aggregate is called Lewy bodies in Parkinson's disease, and neurofibrillary tangles in Alzheimer's disease. Alpha-synuclein and tau have been identified as the major components of the Lewy bodies and the neurofibrillary tangles, respectively.

Another degenerative disease in which accumulations appear in nerve cells is amyotrophic lateral sclerosis, and in this disease, TAR-DNA binding protein of 43 kDa (TDP-43) is accumulated. TDP-43 is a protein that is thought to exhibit cytotoxicity and is known to accumulate in the inclusion body in amyotrophic lateral sclerosis. In addition, it is considered that the TDP-43 aggregate itself has novel cytotoxic effects.

On the other hand, in Alzheimer's disease, a protein called amyloid β protein (Aβ) is known as a protein that accumulates outside of cells. Aβ is thought to weakly interact with tau.

Therefore, at least one type of protein selected from, for example, the group consisting of alpha-synuclein, tau, Aβ, and TDP-43 can be exemplified as a neurodegenerative disease-associated protein in the present invention.

The seed activity-reduced variant protein used in the present invention is a protein comprising a mutation such as a deletion, substitution or addition of one or several amino acids (for example, 1 to 10, preferably 1 to 5 amino acids) (wherein the "amino acid" means an amino acid residue, but it is simply referred to as an "amino acid"; the same applied hereafter) in the amino acid sequence of the PF interaction region of the protein, and it is a protein with reduced seed activity inside or outside a cell.

The amino acid sequence to be mutated in the amino acid sequence of the PF interaction region is not particularly limited, but it is preferably a basic amino acid(s) present in the PF interaction region. In addition, the term "PF interaction region "means a region in which PFs are in contact with each other.

The following aspects are given as mutations of amino acids in the neurodegenerative disease-associated protein.

### (1) Alpha-synuclein

In the case of alpha-synuclein, the PF interaction region is a region ranging from 24 to 64 in the amino acid sequence of alpha-synuclein. Accordingly, alpha-synuclein variants include a variant comprising an amino acid sequence comprising a deletion of one or several basic amino acids from the amino acid sequence of the interaction region, a variant comprising an amino acid sequence comprising a substitution of one or several basic amino acids with non-basic amino acid(s) in the amino acid sequence of the interaction region, and a variant comprising an amino acid sequence comprising an addition of one or several amino acids to the amino acid sequence of the interaction region. These variants have reduced seed activity.

Examples of the aspect of mutation of alpha-synuclein may include an aspect of substitution of at least one of the 43rd lysine (Lys43), the 45th lysine (Lys45), and the 50th histidine (His50) with another amino acid (e.g. a non-basic amino acid ); and an aspect of deletion of at least one of the 43rd lysine (Lys43), the 45th lysine (Lys45), and the 50th histidine (His50) (e.g., SEQ ID No: 18; except for an aspect where, in SEQ ID No: 18, the 43rd, 45th, and 50th amino acids are all wild-type amino acids (lysine, lysine, and histidine, respectively) or basic amino acids).

In the present invention, examples of the alpha-synuclein variant may include a variant in which the 43rd or 45th lysine is substituted with alanine (K43A or K45A) in the amino acid sequence of alpha-synuclein (e.g., SEQ ID No: 2), a variant in which both the 43rd and 45th lysines are substituted with alanines (K43&45A) therein, a variant in which both the 43rd and 45th lysines are substituted with glutamic acids (K43&45E) therein, a variant in which both the 43rd and 45th are deleted (K43&45 delta) therein, and a variant in which the 50th histidine is substituted with alanine (H50A) therein.

### (2) Tau

In the case of tau, the PF interaction region is a region ranging from 329 to 338 in the amino acid sequence of tau. Accordingly, tau variants include, in the amino acid sequence of the interaction region, a variant comprising an amino acid sequence comprising a deletion of one or several basic amino acids, a variant comprising an amino acid sequence comprising a deletion of an amino acid sequence comprising the amino acids shown as PGGG, a variant comprising an amino acid sequence comprising a substitution of one or several basic amino acids with non-basic amino acids, a variant comprising an amino acid sequence comprising a substitution of the amino acids shown as PGGG with non-basic amino acids, and a variant comprising an amino acid sequence comprising an addition of one or several amino acids.

Herein, tau has 6 types of isoforms having a length of 352 to 441 amino acids by selective splicing, and their N-terminal flanking region and microtubule-binding region (MBD) are different depending on each isoform type. The N-terminal flanking region contains 2 types of inserts, N1 and N2, which are encoded by the exon 2 and exon 3 of the tau gene, respectively. When both exons 2 and 3 are deleted by selective splicing, type ON is formed. When only exon 2 is present, the isoform is type 1N, and when both exons 2 and 3 are present, the isoform is type 2N. On the other hand, MBD is classified into 4 regions, R1 to R4, of which the presence or absence of R2 (encoded by exon 10) results in the 4R or 3R isoform.

The region from 329 to 338 in the above-described PF interaction region is a region based on the 4R2N isoform.

The aspect of inserts of the 6 types of isoforms is shown below.

| | |
|---|---|
| Type 3R0N: | R1-R3-R4 |
| Type 3R1N: | N1-R1-R3-R4 |
| Type 3R2N: | N1-N2-R1- R3-R4 |
| Type 4R0N: | R1-R2-R3-R4 |
| Type 4R1N: | N1- R1-R2-R3-R4 |
| Type 4R2N: | N1-N2-R1-R2-R3-R4 |

Accordingly, one aspect of mutation of the amino acid sequence of type 4R2N may be an aspect in which the 329th histidine (His329), the 330th histidine (His330), and the 331st lysine (Lys331) are substituted with other amino acids (e.g., SEQ ID No: 30; except for an aspect where the 329th, 330th, and 331st amino acids are all wild-type amino acids (histidine, histidine, and lysine, respectively) or basic amino acids).

In the present invention, examples of the tau variant may include a variant in which the 329th or 330th histidine is substituted with alanine (H329A or H330A) in the amino acid sequence) of tau (e.g., SEQ ID No: 14, a variant in which the 331st lysine is substituted with alanine (K331A) therein, and a variant in which the 329th and 330th histidines and the 331st lysine are substituted with alanines (H329&H330&K331A) therein.

In the present invention, the basic amino acid to be mutated is at least one of His329, His330, and Lys331, based on the amino acid sequence of the 4R2N isoform. His329, His330 and Lys331 are located in R3 of the MBD. The figures used in these terms His329, His330 and Lys331 indicate their existence positions with respect to the amino acid sequence of the 4R2N isoform.

The amino acid sequence of each isoform is shown below (Table 1 as shown later).

Also for other isoforms other than type 4R2N, the amino acids corresponding to His329, His330 and Lys331 in the insert of R3 can be mutated.

The existence positions corresponding to the above-described His329, His330 and Lys331 in other isoforms other than type 4R2N are as follows.
3R0N: the 240th His, 241st His, and 242nd Lys of SEQ ID No: 4
3R1N: the 269th His, 270th His, and 271st Lys of SEQ ID No: 6
3R2N: the 298th His, 299th His, and 300th Lys of SEQ ID No: 8
4R0N: the 271st His, 272nd His, and 273rd Lys of SEQ ID No: 10
4R1N: the 300th His, 301st His, and 302nd Lys of SEQ ID No: 12

Thus, mutations similar to the above 4R2N mutations can be made to the amino acids in these positions (e.g., SEQ ID No: 20, 22, 24, 26, and 28).

### (3) Aβ

In the case of Aβ, the PF interaction region is a region ranging from 26 to 29 or from 33 to37 in the amino acid sequence of Aβ. Accordingly, Aβ variants include, in the amino acid sequence of the interaction region, a variant comprising an amino acid sequence comprising a deletion of one or several hydrophobic amino acids or basic amino acids, a variant comprising an amino acid sequence comprising a substitution of one or several hydrophobic amino acids or basic amino acids with other amino acids, and a variant comprising an amino acid sequence comprising an addition of one or several amino acids.

One aspect of mutation may be an aspect in which the 34th leucine (Leu34), 36th valine (Val36), and 28th lysine (Lys28) are deleted or substituted with other amino acids (e.g., SEQ ID No: 32; except for an aspect where the 34th, 36th, and 28th amino acids in SEQ ID No: 32 are all wild-type amino acids (leucine, valine, and lysine, respectively). In the present invention, examples of the Aβ variant may include a variant in which the 34th leucine is deleted (delta L34) in the amino acid sequence (e.g., SEQ ID No: 16) of Aβ, a variant in which the 36th valine is deleted therein (delta V36), a variant in which the 28th lysine is substituted with alanine therein (K28A), and a variant in which the 34th leucine and the 36th valine are deleted therein (delta L34&V36).

### 2.2. Nucleic acid encoding variant neurodegenerative disease-associated protein (seed activity-reduced variant protein)

### (1) Acquisition of nucleic acid encoding seed activity-reduced variant protein

The seed activity-reduced variant protein used in the present invention can be obtained by obtaining the information of genes or amino acid sequences from the accession numbers shown in Table 1 and then applying known genetic engineering methods or site-specific mutagenesis based on the obtained information (Sambrook J. et al., Molecular Cloning, A Laboratory Manual (4th edition), Cold Spring Harbor Laboratory Press (2012)). The wild-type gene can be chemically synthesized so that it can be the nucleotide sequence indicated with the sequence number shown in Table 1, or a commercially available wild-type gene can be used.

A nucleic acid (DNA) encoding the variant (seed activity-reduced variant protein) can also be obtained by a known method utilizing, for example, site-specific mutagenesis. Examples of a mutagenesis kit that can be used for the site-specific mutagenesis may include QuikChange Site-Directed Mutagenesis Kit (Stratagene), KOD-Plus-Mutagenesis Kit (Toyobo Co., Ltd.), GenEdit Site-Directed DNA Mutagenesis Kit (Funakoshi), and TaKaRa Site-Directed Mutagenesis System (Mutan-K, Mutan-Super Express Km, etc.: Takara Bio Inc. (Mutan-K, Mutan-Super Express Km, etc.: Takara Bio Inc.).

Moreover, the nucleic acid encoding the above-described protein can also be produced by ordinary chemical synthesis method or biochemical synthesis method. For example, a nucleic acid synthesis method using a DNA synthesizer commonly used as a genetic engineering method, or a gene amplification method using a PCR method or a cloning vector after isolating or synthesizing a nucleotide sequence serving as a template can be used. Thereafter, the nucleic acid obtained as described above is cleaved by restriction enzymes, etc. The thus cleaved DNA fragment of the gene is inserted into an appropriate expression vector, so as to obtain an expression vector containing the gene encoding the protein.

As described above, the protein includes alpha-synuclein, tau, and Aβ. The amino acid sequences of these proteins and the accession numbers of the nucleic acids (genes) encoding these proteins are shown in Table 1.

### [Table 1]

**Table 1**

| Protein | Accession No. | Nucleotide sequence | Amino acid sequence |
|---|---|---|---|
| Alpha-synuclein | NM 000345.4 | SEQ ID No: 1 | SEQ ID No: 2 |
| Tau 3R0N | NM 016841.5 | SEQ ID No: 3 | SEQ ID No: 4 |
| Tau 3R1N | NM 001203251.2 | SEQ ID No: 5 | SEQ ID No: 6 |
| Tau 3R2N | NM 001203252.2 | SEQ ID No: 7 | SEQ ID No: 8 |
| Tau 4R0N | NM 016834.5 | SEQ ID No: 9 | SEQ ID No: 10 |
| Tau 4R1N | NM 001123067.4 | SEQ ID No: 11 | SEQ ID No: 12 |
| Tau 4R2N | NM 005910.6 | SEQ ID No: 13 | SEQ ID No: 14 |
| Aβ (Aβ42) | NM_000484 | SEQ ID No: 15 | SEQ ID No: 16 |

Furthermore, in the present invention, examples of genes encoding the above-described variants (seed activity-reduced variant proteins) may include the following genes.

### < Variants of alpha-synuclein >

(a-1) A gene that encodes a variant alpha-synuclein, comprising a variant amino acid sequence in which at least one of the 43rd, 45th, and 50th amino acids is substituted with another amino acid or is deleted in the amino acid sequence (e.g., SEQ ID No: 2) of alpha-synuclein, and having reduced seed activity functioning as a nucleus of an aggregate of alpha-synuclein.

Examples of the amino acid sequence of the above-described seed activity-reduced variant alpha-synuclein may include the above-described "K43A," "K45A," "K43&45A," "K43&45E," "K43&45 delta," and "H50A."

The nucleotide sequence of the gene encoding the above-described variant alpha-synuclein is as set forth in SEQ ID No: 17, and the amino acid sequence of the variant alpha-synuclein is as set forth in SEQ ID No: 18.
(a-2) A gene that encodes a variant alpha-synuclein, comprising an amino acid sequence in which at least one of the 43rd, 45th, and 50th amino acids is substituted with another amino acid or is deleted, and one or several amino acids other than the 43rd, 45th, and 50th amino acids are deleted, substituted or added, in the amino acid sequence (e.g., SEQ ID No: 2) of alpha-synuclein, and having reduced seed activity functioning as a nucleus of an aggregate of alpha-synuclein.
(a-3) A gene that consists of DNA comprising the nucleotide sequence as set forth in SEQ ID No: 17.
(a-4) A gene that consists of DNA, hybridizing under stringent conditions with DNA consisting of a nucleotide sequence complementary to the DNA comprising the nucleotide sequence as set forth in SEQ ID No: 17, and encoding a variant alpha-synuclein with reduced seed activity functioning as a nucleus of an aggregate of alpha-synuclein.

However, the nucleotide sequences encoding the 43rd amino acid, the 45th amino acid, and the 50th amino acid in the above-described gene that hybridizes under stringent conditions shall always hybridize as a codon of the amino acids after substitution or deletion.

Herein, in the present invention, hybridization can be carried out according to known methods (e.g., Sambrook J. et al., Molecular Cloning, A Laboratory Manual (4th edition) (Cold Spring Harbor Laboratory Press (2012)). Highly stringent conditions are conditions under which so-called specific hybrids are formed and nonspecific hybrids are not formed, for example, conditions under which sodium concentration is 10 mM to 300 mM, preferably 20 mM to 100 mM, and the temperature is 25°C to 70°C, preferably 42°C to 55°C. These stringent conditions can be applied throughout the present description as a whole.

### < Variants of tau >

### (i) Type 4R2N

(b-1-1) A gene that encodes a variant tau, comprising a variant amino acid sequence in which at least one selected from the 329th amino acid, the 330th amino acid, the 331st amino acid, and an amino acid sequence comprising the amino acid sequence shown as PGGG is substituted with another amino acid or is deleted in the amino acid sequence (e.g., SEQ ID No: 14) of 4R2N tau, and having reduced seed activity functioning as a nucleus of an aggregate of tau.

The nucleotide sequences and amino acid sequences of the above-described 4R2N variants are shown in Table 2 later.

In the present invention, the concerned amino acid sequence is preferably the amino acid sequence as set forth in SEQ ID No: 14, in which the 329th and 330th amino acids, histidines, are substituted with alanines, and the 331st lysine is substituted with alanine.

(b-1-2) A gene that encodes a variant tau, comprising an amino acid sequence in which at least one selected from the 329th amino acid, the 330th amino acid, the 331st amino acid, and an amino acid sequence comprising the amino acid sequence shown as PGGG is substituted with another amino acid or is deleted, and one or several amino acids other than the 329th amino acid, the 330th amino acid, the 331st amino acid, and the amino acid sequence comprising the amino acid sequence shown as PGGG are deleted, substituted or added, in the amino acid sequence (e.g., SEQ ID No: 14) of tau, and having reduced seed activity functioning as a nucleus of an aggregate of tau.

(b-1-3) A gene that consists of DNA comprising the nucleotide sequence as set forth in SEQ ID No: 29.

(b-1-4) A gene that consists of DNA, hybridizing under stringent conditions with DNA consisting of a nucleotide sequence complementary to the DNA comprising the nucleotide sequence as set forth in SEQ ID No: 29, and encoding a variant tau with reduced seed activity functioning as a nucleus of an aggregate of tau.

However, the nucleotide sequences encoding the 329th amino acid, the 330th amino acid, the 331st amino acid, and the amino acid sequence comprising the amino acid sequence shown as PGGG in the above-described gene that hybridizes under stringent conditions shall always hybridize as a codon of the amino acids after substitution.

### (ii) Other isoforms other than 4R2N isoform

(b-2-1) A gene that encodes a variant tau, comprising a variant amino acid sequence in which at least one selected from amino acids at positions that correspond to the 329th amino acid, the 330th amino acid, the 331st amino acid and an amino acid sequence comprising the amino acid sequence shown as PGGG in 4R2N, in the amino acid sequence (e.g., SEQ ID No: 4, 6, 8, 10 or 12) of an isoform other than the 4R2N isoform, is substituted with another amino acid or is deleted, and having reduced seed activity functioning as a nucleus of an aggregate of tau.

In the present invention, the concerned amino acid sequence is preferably the amino acid sequence as set forth in SEQ ID No: 4, 6, 8, 10 or 12, in which the 329th and 330th amino acids, histidines, are substituted with alanines, and the 331st lysine is substituted with alanine.

(b-2-2) A gene that encodes a variant tau, comprising an amino acid sequence in which at least one selected from amino acids at positions that correspond to the 329th amino acid, the 330th amino acid, the 331st amino acid and an amino acid sequence comprising the amino acid sequence shown as PGGG in 4R2N, in the amino acid sequence (e.g., SEQ ID No: 14) of the 4R2N isoform, is substituted with another amino acid or is deleted, in the amino acid sequence (e.g., SEQ ID No: 4, 6, 8, 10 or 12) of an isoform other than the 4R2N isoform, and one or several amino acids other than amino acids at positions that correspond to the 329th amino acid, the 330th amino acid, the 331st amino acid and the amino acid sequence comprising the amino acid sequence shown as PGGG are deleted, substituted or added, and having reduced seed activity functioning as a nucleus of an aggregate of tau.

(b-2-3) A gene that consists of DNA comprising the nucleotide sequence as set forth in SEQ ID No: 19, 21, 23, 25 or 27.

(b-2-4) A gene that consists of DNA, hybridizing under stringent conditions with DNA consisting of a nucleotide sequence complementary to the DNA comprising the nucleotide sequence as set forth in SEQ ID No: 19, 21, 23, 25 or 27, and encoding a variant tau with reduced seed activity functioning as a nucleus of an aggregate of tau.

However, the nucleotide sequences encoding amino acids at positions corresponding to the amino acid sequences comprising the 329th amino acid, the 330th amino acid, the 331st amino acid, and the amino acid sequence comprising the amino acid sequence shown as PGGG, in the above-described gene that hybridizes under stringent conditions, shall always hybridize as a codon of the amino acids after substitution.

The amino acid sequences of other isoforms other than 4R2N of tau are shown in Table 1.

Moreover, in other isoforms other than 4R2N, the positions of amino acids corresponding to the 329th amino acid, the 330th amino acid, and the 331st amino acid of 4R2N are as follows.
3R0N: the 240th His, 241st His, and 242nd Lys of SEQ ID No: 4
3R1N: the 269th His, 270th His, and 271st Lys of SEQ ID No: 6
3R2N: the 298th His, 299th His, and 300th Lys of SEQ ID No: 8
4R0N: 271st His, 272nd His, and 273rd Lys of SEQ ID No: 10
4R1N: 300th His, 301st His, and 302nd Lys of SEQ ID No: 12

The nucleotide sequences of genes encoding these variant tau isoforms, and the amino acid sequences of the isoforms are shown in Table 2 below.

### [Table 2]

**Table 2**

| Protein (without seed activity) | Nucleotide sequence | Amino acid sequence |
|---|---|---|
| Variant tau 3R0N | SEQ ID No: 19 | SEQ ID No: 20 |
| Variant tau 3R1N | SEQ ID No: 21 | SEQ ID No: 22 |
| Variant tau 3R2N | SEQ ID No: 23 | SEQ ID No: 24 |
| Variant tau 4R0N | SEQ ID No: 25 | SEQ ID No: 26 |
| Variant tau 4R1N | SEQ ID No: 27 | SEQ ID No: 28 |
| Variant tau 4R2N | SEQ ID No: 29 | SEQ ID No: 30 |

### < Variants of Aβ >

(c-1) A gene that encodes a variant Aβ, comprising a variant amino acid sequence in which at least one of the 34th amino acid, the 36th amino acid and the 28th amino acid is substituted with another amino acid in the amino acid sequence (e.g., SEQ ID No: 16) of Aβ, and having reduced seed activity functioning as a nucleus of an aggregate of Aβ.

In the present invention, the concerned amino acid sequence is preferably the amino acid sequence as set forth in SEQ ID No: 16, in which the 34th amino acid, leucine, is deleted, the 36th amino acid, valine, is deleted, and the 28th amino acid, lysine, is substituted with alanine.

The nucleotide sequence of the gene encoding the variant Aβ is as set forth in SEQ ID No: 31, and the amino acid sequence of the variant Aβ is as set forth in SEQ ID No: 32.

(c-2) A gene that encodes a variant Aβ, comprising an amino acid sequence in which at least one of the 34th amino acid, the 36th amino acid and the 28th amino acid is substituted with another amino acid, and one or several amino acids other than the 34th amino acid, the 36th amino acid and the 28th amino acid are deleted, substituted or added, in the amino acid sequence (e.g., SEQ ID No: 16) of Aβ, and having reduced seed activity functioning as a nucleus of an aggregate of Aβ.

(c-3) A gene that consists of DNA comprising the nucleotide sequence as set forth in SEQ ID No: 31.

(c-4) A gene that consists of DNA, hybridizing under stringent conditions with DNA consisting of a nucleotide sequence complementary to the DNA comprising the nucleotide sequence as set forth in SEQ ID No: 31, and encoding a variant Aβ with reduced seed activity functioning as a nucleus of an aggregate of Aβ.

However, the nucleotide sequences encoding the 34th amino acid, the 36th amino acid and the 28th amino acid in the above-described gene that hybridizes under stringent conditions shall always hybridize as a codon of the amino acids after substitution.

### (2) Construction of expression vector and transformation

In the present invention, the seed activity-reduced variant protein can be obtained by introducing a nucleic acid encoding the variant protein into a vector to construct an expression vector (recombinant vector), then introducing the expression vector into a host, and then culturing the host, as described below.

The vector for insertion of the nucleic acid (DNA, etc.) encoding the variant protein of the present invention is not particularly limited, as long as it can replicate in a host. Examples of the vector may include plasmid DNA, phage DNA, and viruses. Examples of the plasmid DNA may include plasmids derived from *Escherichia coli,* plasmids derived from *Bacillus subtilis,* and plasmids derived from yeasts. An example of the phage DNA may be lambda phage. Also, examples of the viruses that can be used herein as vectors may include adenovirus (e.g., adeno-associated virus) and retrovirus.

In the present invention, to the expression vector, promoters, DNA, and as desired, cis-elements such as an enhancer, and splicing signals, poly-A addition signals, ribosome binding sequences (SD sequences), selection marker genes, reporter genes, etc. can be ligated.

The above-described expression vector is introduced into a host to create a transformant, which is then used in the expression of a gene of interest. At this time, the host is not particularly limited, as long as it can express the gene of interest. Examples of the host may include bacteria such as *Escherichia coli* and *Bacillus subtilis,* yeasts such as *Saccharomyces cerevisiae,* and mammalian cells such as COS cells and CHO cells. Insect cells, insects, rodents (a mouse, a rat, a guinea pig, etc.), and non-human mammals (a goat, a bovine, etc.) can also be used as hosts. Methods of introducing the recombinant vector into the host are well known, and examples of the introduction method may include an electroporation method, a liposome method, a spheroplast method, and a lithium acetate method (Sambrook J. et al. Manual (4th edition), Cold Spring Harbor Laboratory Press (2012)).

### (3) Collection and purification of seed activity-reduced variant protein or aggregate thereof

The above-described transformant is cultured or reared, and a seed activity-reduced variant protein of interest is then collected from the culture product or non-human mammal. The term "culture product" means all of (a) a culture supernatant, and (b) cultured cells or a cultured cell mass, or a crushed product thereof.

When the seed activity-reduced variant protein is produced in the cell mass or in the cells after completion of the culture, the variant protein is extracted by crushing the cell mass or the cells. On the other hand, when the variant protein is produced outside the cell mass or outside the cells, the culture medium is directly used, or the cell mass or the cells are removed by centrifugation, etc. When the host is a mammal (rodents, a goat, a bovine etc.), the seed activity-reduced variant protein is collected from body fluids (serum, saliva, etc.), secretions (milk, etc.), or tissues (brain, etc.). Thereafter, by using general biochemical methods used for isolation and purification of proteins, such as ammonium sulfate precipitation, gel filtration, ion exchange chromatography, affinity chromatography, hydrophobic chromatography, reversed phase chromatography, etc., alone or in combination as appropriate, a variant protein of interest can be isolated and purified.

The purified seed activity-reduced variant protein can be aggregated by shaking a solution containing the protein, for example, at 37°C. The aggregate obtained by aggregation is collected by ultracentrifugation, etc., is then suspended in an appropriate amount of buffer solution, and is then used for cell introduction, or as a pharmaceutical composition or a vaccine.

### 2.3. Antibody against aggregate of seed activity-reduced variant protein

The "antibody" of the present invention means an antibody specifically binding to an aggregate of the above-described variant protein (a seed activity-reduced aggregate), or a fragment thereof. The present antibody may be either a polyclonal antibody or a monoclonal antibody.

The antibody of the present invention also includes an antibody that binds to an antigenic determinant (epitope) to which the antibody of the present invention binds. The antigenic determinant is a region of, the entire seed activity-reduced variant protein or a part of the aggregate, and examples thereof may include the interaction region of two molecules of protofilaments (PF) in the amino acid sequence of the neurodegenerative disease-associated proteins, and regions other than the PF.

The antibody of the present invention may be either a polyclonal antibody or a monoclonal antibody, and their antibody fragments are also included. In addition, the antibody of the present invention also includes a chimeric antibody, a human reshaped antibody, and a humanized antibody. Methods for producing antibodies are known in the present technical field.

### (1) Preparation of polyclonal antibody

In order to prepare a polyclonal antibody, a seed activity-reduced variant proteins or a partial peptide is administered alone or together with a carrier and a diluent to a non-human mammal such as, for example, a rabbit, a dog, a guinea pig, a mouse, a rat, a goat, etc., so that the non-human mammal is immunized. The dosage of an antigen per animal, the dosage of an adjuvant when it is used, the type of the adjuvant, an immunization site, interval of immunization, etc. are well known.

The measurement of an antibody titer in serum is also well known in the present technical field, and it can be performed by ELISA, EIA, RIA, etc. After confirming that the antibody titer has risen sufficiently, whole blood can be collected, and the antibody can be separated and purified by a commonly used method. Separation and purification can be carried out by appropriately selecting a method from among known methods such as an ammonium sulfate salting method, ion exchange chromatography, gel filtration chromatography and affinity chromatography, or by combining these methods.

### (2) Preparation of monoclonal antibody

Methods of preparing monoclonal antibodies are also well known. For example, collection of antibody-producing cells, cell fusion between antibody-producing cells and myeloma cells to obtain hybridomas, selection and cloning of hybridomas, and collection of monoclonal antibodies, etc. can be performed by methods well known to those skilled in the art.

In addition, in the present invention, the epitope (antigenic determinant) of the antibody is not limited, as long as it is at least a part of a protein derived from the antigenic aggregate that is an antigen. The antibody of the present invention includes an antibody that binds to the site (e.g., epitope) to which the present antibody binds, for example, an antibody that binds to the site to which the antibody produced by the hybridoma binds.

### (3) Preparation of genetic recombinant antibody

One preferred aspect of the antibody of the present invention may be a genetic recombinant antibody. Examples of such a genetic recombinant antibody may include, but are not limited to, a chimeric antibody, a human reshaped antibody, and a humanized antibody.

A chimeric antibody (i.e., a human-type chimeric antibody) is an antibody in which the variable region of a mouse-derived antibody is linked (connected) to a human-derived constant region (see Proc. Natl. Acad. Sci. U.S.A. 81, 6851-6855 (1984), etc.), and in the case of preparing such a chimeric antibody, it can be constructed by genetic recombination technology so as to obtain such a linked antibody.

In the case of preparing a human reshaped antibody, what is called CDR grafting (CDR transplantation) can be adopted. Such a method of preparing a human reshaped antibody is well known in the present technical field (see Nature, 321, 522-525 (1986); J. Mol. Biol., 196, 901-917 (1987); Queen C et al., Proc. Natl. Acad. Sci. USA, 86: 10029-10033 (1989); Japanese Patent No.2828340; etc.).

A human antibody (complete human antibody) generally has the same structure as a human antibody in terms of a hyper variable region that is an antigen binding site of a variable region (V region), other parts of the V region, and a constant region. The technique of preparing a human antibody is known, and regarding gene sequences common in humans, a method of preparing the gene sequences according to genetic engineering technique has been established. The human antibody can be obtained, for example, by a method of using human antibody-producing mice having human chromosomal fragments comprising the genes of the heavy chain (H chain) and light chain (L chain) of a human antibody (see Tomizuka, K.et al., Nature Genetics, (1977) 16, 133-143; Kuroiwa, Y. et. al., Nuc. Acids Res., (1998) 26, 3447-3448; Yoshida, H. et. al., Animal Cell Technology: Basic and Applied Aspects, (1999) 10, 69-73 (Kitagawa, Y, Matuda, T. and Iijima, S. eds.), Kluwer Academic Publishers; Tomizuka, K. et. al., Proc. Natl. Acad. Sci. USA, (2000) 97, 722-727, etc.) or by a method of obtaining a phage display-derived human antibody selected from a human antibody library (see Wormstone, I. M. et. al., Investigative Ophthalmology & Visual Science., (2002) 43 (7), 2301-8; Carmen, S. et. al., Briefings in Functional Genomics and Proteomics, (2002) 1 (2), 189-203; Siriwardena, D. et. al., Opthalmology, (2002) 109 (3), 427-431, etc.).

### (4) Preparation of antibody fragments

Examples of fragments of an antibody against the seed activity-reduced variant protein used in the present invention may include Fab, Fab', F(ab')2, Fv, diabody (dibodies), dsFv, and scFv (single chain Fv). The aforementioned antibody fragments can be obtained by cleaving the antibody of the present invention by various types of proteases, according to purpose.

For example, Fab is obtained by treating the antibody molecule with papain, and F(ab')2 is obtained by treating the antibody molecule with pepsin. Fab' is obtained by cleaving the disulfide bond in the hinge region of the aforementioned F(ab')2. In the case of scFv, cDNAs encoding the heavy chain variable region (H chain V region) and the light chain variable region (L chain V region) of the antibody are obtained, and the DNA encoding scFv is constructed. This DNA is inserted into an expression vector, and the expression vector is introduced into a host organism for expression, so as to produce scFv.

In the case of diabody, cDNAs encoding the H chain V region and the L chain V region of the antibody are obtained, and the DNA encoding the diabody is constructed so that the length of the amino acid sequence of the peptide linker becomes 8 residues or less. This DNA is inserted into an expression vector, and the expression vector is introduced into a host organism for expression, so as to produce a diabody. In case of dsFv, cDNAs encoding the H chain V region and the L chain V region of the antibody are obtained, and the DNA encoding dsFv is constructed. This DNA is inserted into an expression vector, and the expression vector is introduced into the host organism for expression, so as to produce dsFv.

### 2.4. Pharmaceutical composition, and therapeutic or preventive method for neurodegenerative disease

The pharmaceutical composition of the present invention comprises, as an active ingredient, the seed activity-reduced variant protein of the present invention, a nucleic acid encoding the variant protein, or a vector containing the nucleic acid. The present pharmaceutical composition is effective for prevention or treatment of diseases such as related neurodegenerative diseases including alpha-synucleinopathy (dementia with Lewy bodies, Parkinson's disease, and multiple system atrophy), Alzheimer's disease, and motor neuron disease. The seed activity-reduced variant protein of the present invention is applied as a pharmaceutical composition for protein therapy, and the nucleic acid encoding the variant protein is applied as a pharmaceutical composition for gene therapy.

The seed activity-reduced variant protein of the present invention, or the nucleic acid encoding the variant protein, or the vector containing the nucleic acid can be administered alone or together with a pharmaceutically acceptable carrier or diluent, etc. to a subject in need of treatment or prevention (e.g., a patient with neurodegenerative disease), and can be administered in once or several divided doses.

Neurodegenerative disease, which is the target of treatment or prevention in the present invention, means a disease in which the phenomenon of neurodegeneration, in which nerve cells die without any apparent cause such as trauma or bacterial infection, is observed. Examples of such neurodegenerative disease may include alpha-synucleinopathy (dementia with Lewy bodies, Parkinson's disease, and multiple system atrophy) and Alzheimer's dementia. In addition to these diseases, other examples of the neurodegenerative disease may include Huntington's disease, triplet repeat disease, amyotrophic lateral sclerosis, Creutzfeldt-Jakob disease, Gerstmann-Straussler syndrome, mad cow disease, spinal and bulbar muscular atrophy, spinocerebellar ataxia, dentatorubral-pallidoluysian atrophy, FTDP-17, progressive supranuclear palsy, corticobasal degeneration, and Pick's disease.

Examples of the "pharmaceutically acceptable carrier" used herein may include an excipient, a diluent, a bulking agent, a disintegrant, a stabilizer, a preservative, a buffer, an emulsifier, an aromatic agent, a coloring agent, a sweetening agent, a viscosity agent, a taste masking agent, a solubilizing agent, and other additives. By using one or more of such carriers, the present pharmaceutical composition can be prepared in the form of a tablet, a pill, a powdery agent, a granule, an injection, a liquid agent, a capsule, a troche, an elixir, a suspension, an emulsion or syrup, etc. These pharmaceutical compositions can be administered orally or parenterally. The carriers used herein also include carriers used in protein preparations such as lipid nanoparticles (LNPs) or for gene therapy.

In the case of oral administration, various excipients such as microcrystalline cellulose, sodium citrate, calcium carbonate, dipotassium phosphate and glycine can be used together with disintegrants, binders, etc. Examples of the disintegrants may include starch, alginic acid, and certain types of silicic acid double salts. Examples of the binders may include polyvinylpyrrolidone, sucrose, gelatin, and gum Arabic. Lubricants such as magnesium stearate, sodium lauryl sulfate and talc are very effective for tablet formation. When an aqueous suspension or an elixir is prepared for oral administration, it can be used in combination with an emulsifier and a suspending agent, as necessary, and it can be used together with water, ethanol, propylene glycol, glycerin, etc., and a diluent that is a combination thereof.

Another form for parenteral administration may be an injection containing one or more active substances, which is formulated according to an ordinary method.

In the case of an injection, for example, it can be produced by dissolving or suspending the present pharmaceutical composition in a pharmaceutically acceptable carrier such as a normal saline or a commercially available distilled water for injection, to achieve a predetermined concentration. The concentration of the active ingredient in the carrier for each pharmaceutical composition is as follows.
- Seed activity-reduced variant protein: The dosage range can be 10 µg/Kg to 100 mg/Kg, preferably 100 µg/Kg to 50 mg/Kg, with intervals of from once a day, to several days to several months.
- Nucleic acid encoding seed activity-reduced variant protein: The dosage (effective dose) is desirably adjusted according to the type of nucleic acid contained, the dosage form, etc. However, the dosage of the nucleic acid encoding the seed activity-reduced variant protein per adult is 0.1 mg to 100 g/human per day, preferably 1 mg to 5 g/human per day, and can be administered with intervals of from once a day, to several days to several months. In the case of administering gene therapy virus, for example, the effective dose is 1 × 10¹⁰ to 1 × 10¹⁵ genome copies. This dose can be administered from once to several times.

The thus produced injection can be administered to a human patient in need of treatment or prevention at a rate of 1 µg to 500 mg per kg of body weight per dose, preferably 100 µg to 50 mg, once to several times per day. However, the dosage is not limited to this range and may vary depending on the patient's body weight, symptoms, and individual routes of administration. Since a difference in the sensitivity of the patient to be treated to the drug, the way of prescription of the drug, the administration period and the administration intervals may also cause fluctuation in the dosage, a dosage lower than the lower limit in the above-described range may be appropriate in some cases, while a higher dosage may be necessary in other cases.

Examples of the administration form may include intravenous, subcutaneous, intradermal, and intramuscular injections, with intravenous injection being the preferred form. In addition, in some cases, the injection can also be prepared as a non-aqueous diluent (e.g., propylene glycol, polyethylene glycol, vegetable oils such as olive oil, alcohols such as ethanol, etc.), a suspension, or an emulsion. Sterilization of such an injection can be achieved by filter sterilization through a bacteria-retaining filter, by addition of a sterilizing agent, or by irradiation. The injection can be produced as a form that is to be prepared at time of use. That is, a sterile solid composition can be prepared by a freeze-drying method, etc., and before use, the sterile solid composition can be dissolved in sterile distilled water for injection or in other solvents.

### 2.5. Vaccine

The seed activity-reduced aggregate of the present invention is used as a vaccine. The target diseases are the same as those described in the section of "Pharmaceutical composition," and as a typical example, the present seed activity-reduced aggregate can be used for the treatment or prevention of alpha-synucleinopathy (dementia with Lewy bodies, Parkinson's disease, and multiple system atrophy), and Alzheimer's dementia.

The vaccine of the present invention can be used for the prevention or treatment of the onset of alpha-synucleinopathy (a general term for diseases involving accumulation of aS). The vaccine of the present invention is characterized in that an aggregate with reduced seed activity (i.e., low pathogenicity) is utilized as a vaccine. In addition, regarding not only aS, but also tau and A beta, an aggregate thereof with reduced seed activity can be used as a vaccine.

Thus, in the present invention, it is possible to use a mixture of vaccines. This makes it possible to prevent or treat multiple neurodegenerative diseases with a single vaccination. For example, a double combined vaccine consisting of an A beta vaccine (Aβ aggregate) and a tau vaccine (tau aggregate) is effective for the prevention or treatment of Alzheimer's disease, and a double combined vaccine consisting of a tau vaccine and an aS vaccine (aS aggregate) can simultaneously prevent or treat tauopathy and alpha-synucleinopathy. Further, a combined vaccine consisting of these three types of vaccines can also simultaneously prevent or treat tauopathy including Alzheimer's disease, and alpha-synucleinopathy.

In a preferred aspect of the present invention, the seed activity-reduced aggregate is combined with a pharmaceutically acceptable carrier, and examples of such a carrier may include KLH (keyhole limpet hemocyanin), albumin-binding protein, bovine serum albumin, dendrimer, and peptide linker. Furthermore, the vaccine composition can be formulated together with an adjuvant. Examples of the adjuvant may include aluminum hydroxide, aluminum phosphate, calcium phosphate, saponin, emulsion (e.g. Freund's adjuvant), and liposome.

The vaccines of the present invention can be administered by means of administration methods or delivery devices, such as intradermal, intravenous, intraperitoneal, intramuscular, intranasal, oral, and subcutaneous administrations.

Typically, the present vaccine may comprise the seed activity-reduced aggregate of the present invention at 100 ng to 200 µg, for example, 1 µg to 10 mg, or 1 mg to 100 mg. Regarding the administration method of the vaccine and the vaccine formulation, there can be referred to the section "Pharmaceutical composition" as described above.

### 3. Variant neurodegenerative disease-associated protein having seed activity

### 3.1. Mutation of amino acids, and aggregates

The variant neurodegenerative disease-associated protein of the present invention is a protein comprising an amino acid sequence comprising a substitution of one or several hydrophobic amino acids or basic amino acids with other basic amino acids in the amino acid sequence of the interaction region of two molecules of protofilaments (PF) in the amino acid sequence of a neurodegenerative disease-associated protein (provided that when a basic amino acid(s) are substituted, they are substituted with other basic amino acid(s) having a higher charge than the concerned basic amino acids), or comprising an amino acid sequence comprising an addition or a deletion of one or several basic amino acids to/from the PF interaction region, and having seed activity that functions as a nucleus of an aggregate of the neurodegenerative disease-associated protein that is 71% or more compared with the seed activity of a wild-type neurodegenerative disease-associated protein.

In the present description, the phenomenon that the seed activity of the concerned variant protein has an activity of 71% or more compared with the seed activity of a wild-type neurodegenerative disease-associated protein is referred to as "to have seed activity," and the variant neurodegenerative disease-associated protein having the seed activity is also referred to as a "seed activity-possessing variant protein," or a "seed activity-possessing variant." Moreover, an aggregate, in which the variant neurodegenerative disease-associated protein having seed activity is aggregated, is also referred to as a "seed activity-possessing aggregate." Herein, the variant protein having an activity of 71% or more includes variants that maintain essentially the same activity (71% to 100%) compared with the wild-type protein, and variants with an activity of more than 100% (e.g., 1.1-fold (110%), 1.2-fold (120%), 1.3-fold (130%), 1.4-fold (140%), 1.5-fold (150%), 2-fold (200%), and 3-fold (300%)), which have higher activity than that of the wild-type protein.

The amino acid sequence present in the PF interaction region is not particularly limited, and it is preferably a hydrophobic amino acid(s) or a basic amino acid(s) present in the PF interaction region.

The following aspects are given as mutations of amino acids in the neurodegenerative disease-associated protein.

### (1) Alpha-synuclein

In the case of alpha-synuclein, the PF interaction region is a region ranging from amino acid residues at positions 24 to 64 in the amino acid sequence of alpha-synuclein. Accordingly, alpha-synuclein variants include a variant comprising an amino acid sequence comprising a substitution of one or several hydrophobic amino acids or basic amino acids with other basic amino acid(s) in the amino acid sequence of the PF interaction region, and a variant comprising an amino acid sequence comprising an addition or a deletion of one or several amino acids to/from the amino acid sequence of the PF interaction region. However, when a basic amino acid is substituted with another basic amino acid, it is substituted with another basic amino acid having a higher charge than the basic amino acid before substitution. The basic amino acids are arginine, lysine and histidine, and the order of the amino acids is arginine, lysine, and histidine in descending order of charge.

Therefore, if the target amino acid to be substituted is lysine, it is substituted with arginine. These variants are seed activity-possessing variants.

The aspect of mutation is, for example, an aspect of substitution of the 43rd lysine (Lys43), the 45th lysine (Lys45) and the 50th histidine (His50) with other amino acids (e.g. SEQ ID No: 34; except for an aspect where, in SEQ ID No: 34, the 43rd, 45th, and 50th amino acids are all wild-type amino acids (lysine, lysine, and histidine, respectively) or non-basic amino acids).

In the present invention, examples of the alpha-synuclein variant may include a variant in which the 43rd or 45th lysine is substituted with arginine (K43R or K45R) in the amino acid sequence (e.g., SEQ ID No: 2) of alpha-synuclein, and a variant in which both the 43rd and 45th lysines are substituted with arginines (K43&45R) therein. Moreover, another example of the alpha-synuclein variant may be a variant in which the 50th histidine is substituted with arginine or lysine (H50R or H50K).

### (2) Tau

In the case of tau, the PF interaction region is a region ranging from amino acid residues at positions 329 to 338 in the amino acid sequence of tau. Accordingly, tau variants include, in the amino acid sequence of the PF interaction region, a variant comprising an amino acid sequence comprising a substitution of one or several basic amino acids with other basic amino acids, a variant comprising an amino acid sequence comprising a substitution of the amino acids shown as PGGG with basic amino acids, and a variant comprising an amino acid sequence comprising an addition or a deletion of one or several basic amino acids. However, when a basic amino acid is substituted with another basic amino acid, it is substituted with another basic amino acid having a higher charge than the basic amino acid before substitution.

One aspect of mutation may be an aspect in which the 329th histidine (His329), the 330th histidine (His330), and the 331st lysine (Lys331) are substituted with other amino acids (e.g., SEQ ID No: 46; except for an aspect where the 329th, 330th, and 331st amino acids are all wild-type amino acids (histidine, histidine, and lysine, respectively) or non-basic amino acids).

In the present invention, examples of the tau variant may include a variant in which the 329th or 330th histidine is substituted with arginine or lysine (H329R, H329K, K330R or H330K) in the amino acid sequence (e.g., SEQ ID No: 14) of the 4R2N tau, a variant in which the 331st lysine is substituted with arginine (K331R) therein, and a variant in which the 329th and 330th histidines and the 331st lysine are substituted with arginines (H329&H330&K331R).

There are 6 isoforms of tau, and the amino acid sequences of the other tau isoforms other than the 4R2N tau are shown in Table 1. In addition, the positions of amino acids corresponding to the 329th, 330th and 331st amino acids of the 4R2N type in the isoforms other than the 4R2N type are as described above.

The correspondence among the above-described His329, His330 and Lys331 isoforms, or the correspondence among the 329th, 330th and 331st in SEQ ID Nos: 4, 6, 8, 10, 12 and 14, is also as described above.

### (3) Aβ

In the case of Aβ, the PF interaction region is a region ranging from the amino acid residues at positions 26 to 29 or from the amino acid residues at positions 33 to37 in the amino acid sequence of Aβ. Accordingly, Aβ variants include, in the amino acid sequence of the PF interaction region, a variant comprising an amino acid sequence comprising a substitution of one or several hydrophobic amino acids with basic amino acids, a variant comprising an amino acid sequence comprising a substitution of one or several basic amino acids with other basic amino acids, and a variant comprising an amino acid sequence comprising an addition of one or several basic amino acids. Herein, when the concerned basic amino acid(s) are substituted with other basic amino acids, they are substituted with other basic amino acids having a higher charge than the concerned basic amino acid(s).

One aspect of mutation may be an aspect in which the 34th leucine (Leu34), 36th valine (Val36), and 28th lysine (Lys28) are substituted with other basic amino acids. Examples of the Aβ variant may include a variant in which the 34th leucine is substituted with arginine (L34R) in the amino acid sequence (e.g., SEQ ID No: 16) of Aβ, a variant in which the 36th valine is substituted with arginine (V36R) therein, a variant in which the 28th lysine is substituted with arginine (K28R) therein, and a variant in which the 34th leucine is substituted with arginine, the 36th valine is substituted with arginine, and the 28th lysine is substituted with arginine (L34& V36&K28R) therein.

### 3.2. Nucleic acid encoding seed activity-possessing variant protein, and vector and transformant

The seed activity-possessing variant protein used in the present invention can be obtained by obtaining the information of genes or amino acid sequences from the accession numbers shown in Table 1 and then applying known genetic engineering methods or site-specific mutagenesis based on the obtained information (Sambrook J. et al., Molecular Cloning, A Laboratory Manual (4th edition), Cold Spring Harbor Laboratory Press (2012)). The wild-type gene can be chemically synthesized so that it can be the nucleotide sequence indicated with the sequence number shown in Table 1, or a commercially available wild-type gene can be used.

### < Variants of alpha-synuclein >

(a-1) A gene that encodes a variant alpha-synuclein, comprising a variant amino acid sequence in which at least one of the 43rd, 45th, and 50th amino acids is substituted with another amino acid in the amino acid sequence (e.g., SEQ ID No: 2) of alpha-synuclein, and having seed activity functioning as a nucleus of an aggregate of alpha-synuclein.

In the present invention, the concerned amino acid sequence is preferably the amino acid sequence as set forth in SEQ ID No: 2, in which the 43rd and 45th amino acids, lysines, are substituted with arginines, and the 50th histidine is substituted with arginine.

The nucleotide sequence of the gene encoding the above-described variant alpha-synuclein is as set forth in SEQ ID No: 33, and the amino acid sequence of the variant alpha-synuclein is as set forth in SEQ ID No: 34.

(a-2) A gene that encodes a variant alpha-synuclein, comprising an amino acid sequence in which at least one of the 43rd, 45th, and 50th amino acids is substituted with another amino acid, and one or several amino acids other than the 43rd, 45th, and 50th amino acids are deleted, substituted or added, in the amino acid sequence (e.g., SEQ ID No: 2) of alpha-synuclein, and having seed activity functioning as a nucleus of an aggregate of alpha-synuclein.

(a-3) A gene that consists of DNA comprising the nucleotide sequence as set forth in SEQ ID No: 34.

(a-4) A gene that consists of DNA, hybridizing under stringent conditions with DNA consisting of a nucleotide sequence complementary to the DNA comprising the nucleotide sequence as set forth in SEQ ID No: 34, and encoding a variant alpha-synuclein having seed activity functioning as a nucleus of an aggregate of alpha-synuclein.

However, the nucleotide sequences encoding the 43rd amino acid, the 45th amino acid, and the 50th amino acid in the above-described gene that hybridizes under stringent conditions shall always hybridize as a codon of the amino acids after substitution.

### < Variants of tau >

### (i) Type 4R2N

(b-1-1) A gene that encodes a variant tau, comprising a variant amino acid sequence in which at least one of the 329th amino acid, the 330th amino acid and the 331st amino acid is substituted with another amino acid in the amino acid sequence (e.g., SEQ ID No: 14) of tau, and having seed activity functioning as a nucleus of an aggregate of tau.

In the present invention, the concerned amino acid sequence is preferably the amino acid sequence as set forth in SEQ ID No: 14, in which the 329th and 330th amino acids, histidines, are substituted with lysines or arginines, and the 331st lysine is substituted with arginine.

(b-1-2) A gene that encodes a variant tau, comprising an amino acid sequence in which at least one of the 329th amino acid, the 330th amino acid and the 331st amino acid is substituted with another amino acid in the amino acid sequence (e.g., SEQ ID No: 14) of tau, and one or several amino acids other than the 329th amino acid, the 330th amino acid and the 331st amino acid are deleted, substituted or added, and having seed activity functioning as a nucleus of an aggregate of tau.

(b-1-3) A gene that consists of DNA comprising the nucleotide sequence as set forth in SEQ ID No: 45.

(b-1-4) A gene that consists of DNA, hybridizing under stringent conditions with DNA consisting of a nucleotide sequence complementary to the DNA comprising the nucleotide sequence as set forth in SEQ ID No: 45, and encoding a variant tau having seed activity functioning as a nucleus of an aggregate of tau.

However, the nucleotide sequences encoding the 329th amino acid, the 330th amino acid and the 331st amino acid in the above-described gene that hybridizes under stringent conditions shall always hybridize as a codon of the amino acids after substitution.

### (ii) Other isoforms other than 4R2N isoform

(b-2-1) A gene that encodes a variant tau, comprising a variant amino acid sequence in which at least one of amino acids at positions corresponding to the 329th amino acid, the 330th amino acid and the 331st amino acid in the amino acids of 4R2N type in the amino acid sequence (e.g., SEQ ID No: 4, 6, 8, 10 or 12) of tau, is substituted with another amino acid, and having seed activity functioning as a nucleus of an aggregate of tau.

In the present invention, the concerned amino acid sequence is preferably the amino acid sequence as set forth in SEQ ID No: 4, 6, 8, 10 or 12, in which histidines at positions corresponding to the 329th and 330th amino acids, are substituted with lysines or arginines, and lysine at position corresponding to the 331st amino acid is substituted with arginine.

(b-2-2) A gene that encodes a variant tau, comprising an amino acid sequence in which at least one of amino acids at positions corresponding to the 329th amino acid, the 330th amino acid and the 331st amino acid in the amino acids of 4R2N type in the amino acid sequence (e.g., SEQ ID No: 4, 6, 8, 10 or 12) of tau is substituted with another amino acid, and one or several amino acids other than the 329th amino acid, the 330th amino acid and the 331st amino acid are deleted, substituted or added, and having seed activity functioning as a nucleus of an aggregate of tau.

(b-2-3) A gene that consists of DNA comprising the nucleotide sequence as set forth in SEQ ID No: 35, 37, 39, 41 or 43.

(b-2-4) A gene that consists of DNA, hybridizing under stringent conditions with DNA consisting of a nucleotide sequence complementary to the DNA comprising the nucleotide sequence as set forth in SEQ ID No: 35, 37, 39, 41 or 43, and encoding a variant tau having seed activity functioning as a nucleus of an aggregate of tau.

However, the nucleotide sequences encoding amino acids at positions corresponding to the 329th amino acid, the 330th amino acid and the 331st amino acid in the above-described gene that hybridizes under stringent conditions shall always hybridize as a codon of the amino acids after substitution.

In other isoforms other than 4R2N type, the positions of amino acids corresponding to the 329th amino acid, the 330th amino acid and the 331st amino acid of 4R2N type are as described above.

The nucleotide sequences of genes encoding these variant tau isoforms, and the amino acid sequences of the isoforms are shown in Table 3 below.

### [Table 3]

**Table 3**

| Protein (with seed activity)) | Nucleotide sequence | Amino acid sequence |
|---|---|---|
| Variant tau 3R0N | SEQ ID No: 35 | SEQ ID No: 36 |
| Variant tau 3R1N | SEQ ID No: 37 | SEQ ID No: 38 |
| Variant tau 3R2N | SEQ ID No: 39 | SEQ ID No: 40 |
| Variant tau 4R0N | SEQ ID No: 41 | SEQ ID No: 42 |
| Variant tau 4R1N | SEQ ID No: 43 | SEQ ID No: 44 |
| Variant tau 4R2N | SEQ ID No: 45 | SEQ ID No: 46 |

### < Variants of Aβ >

(c-1) A gene that encodes a variant Aβ, comprising a variant amino acid sequence in which at least one of the 34th amino acid, the 36th amino acid and the 28th amino acid is substituted with another amino acid in the amino acid sequence (e.g., SEQ ID No: 15) of Aβ, and having seed activity functioning as a nucleus of an aggregate of Aβ.

In the present invention, the concerned amino acid sequence is preferably the amino acid sequence as set forth in SEQ ID No: 15, in which the 34th amino acid, leucine, is substituted with arginine, the 36th amino acid, valine, is substituted with arginine, and the 28th amino acid, lysine, is substituted with arginine.

The nucleotide sequence of the gene encoding the variant Aβ is as set forth in SEQ ID No: 47, and the amino acid sequence of the variant Aβ is as set forth in SEQ ID No: 48.

(c-2) A gene that encodes a variant Aβ, comprising an amino acid sequence in which at least one of the 34th amino acid, the 36th amino acid and the 28th amino acid is substituted with another amino acid, and one or several amino acids other than the 34th amino acid, the 36th amino acid and the 28th amino acid are deleted, substituted or added, in the amino acid sequence (e.g., SEQ ID No: 15) of Aβ, and having seed activity functioning as a nucleus of an aggregate of Aβ.

(c-3) A gene that consists of DNA comprising the nucleotide sequence as set forth in SEQ ID No: 47.

(c-4) A gene that consists of DNA, hybridizing under stringent conditions with DNA consisting of a nucleotide sequence complementary to the DNA comprising the nucleotide sequence as set forth in SEQ **ID** No: 47, and encoding a variant Aβ having seed activity functioning as a nucleus of an aggregate of Aβ.

However, the nucleotide sequences encoding the 34th amino acid, the 36th amino acid and the 28th amino acid in the above-described gene that hybridizes under stringent conditions shall always hybridize as a codon of the amino acids after substitution.

With regard to production of a nucleic acid encoding the seed activity-possessing variant protein, and a vector and a transformant, there can be referred to the above section "2. Variant neurodegenerative disease-associated protein with reduced seed activity."

### 3.3. Neurodegenerative disease animal model or cell model

A cell model comprising an aggregate of the seed activity-possessing variant protein of the present invention induces cell death. This is apparent from the fact that, when the above cells are cultured for a certain period of time, the cells show obvious morphological changes compared with cells into which the protein aggregate is not introduced, and also from the fact that the number of cells decreases. Therefore, the cell of the present invention can be used as a cell model for neurodegenerative disease.

The present invention also provides a non-human animal comprising the seed activity-possessing aggregate. The non-human animal of the present invention can be used as an animal model for neurodegenerative disease. With regard to the type of an animal subjected to the animal model and introduction of the seed activity-possessing variant protein or the aggregate of the protein into the cell or the animal, there can be referred to the above section "2.2. Nucleic acid encoding variant neurodegenerative disease-associated protein (seed activity-reduced variant protein)."

In order to confirm that cell death is induced in the aggregate-accumulating cell model, a cell death assay can be used, for example. The cell death assay method is not particularly limited, and a lactate dehydrogenase leakage assay is applied, for example.

### 3.4. Screening method and kit for neurodegenerative disease drug

The screening method of the present invention is characterized in that a candidate substance (test substance) is allowed to come into contact with cells or a non-human mammal into which the above-described seed activity-possessing variant protein or an aggregate thereof has been introduced. Thereby, it becomes possible to screen a substance that suppresses intracellular accumulation of a protein aggregate, and it also becomes possible to screen a therapeutic agent for neurodegenerative disease.

The target diseases of the drugs to be screened can be referred to the diseases described in "2.4. Pharmaceutical composition, and therapeutic or preventive method for neurodegenerative disease."

The term "contact" means that cells or an animal, into which the seed activity-possessing variant protein or an aggregate thereof has been introduced, and a candidate substance (test substance) are allowed to exist in an identical environment, reaction system or culture system, and examples of the contact may include adding a candidate substance to a cell culture vessel, mixing cells with a candidate substance, culturing cells in the presence of a candidate substance, administering a candidate substance to a non-human mammal.

In a preferred aspect of the present invention, when the neurodegenerative diseases are Parkinson's disease, dementia with Lewy bodies and multiple system atrophy, it is preferable to use alpha-synuclein-introduced cells as fibrotic structures. In this case, the candidate substance is allowed to come into contact with alpha-synuclein-accumulated nerve cells, and the index values or properties that correlate with the target disease in the cells allowed to come into contact with the candidate substance are compared with those in the control cells. Then, based on the results of this comparison, substances that suppress the intracellular accumulation of alpha-synuclein, or substances that alleviated or eliminate the symptoms of Parkinson's disease can be screened. The index values or properties that correlate with the target disease are the following. Only one of these index values or properties may be adopted, or two or more types of the index values or properties may be used in combination.
- Parkinson's disease: the presence or absence of alpha-synuclein accumulation, the presence or absence of the appearance of Lewy bodies, the presence or absence of reactivity by anti-ubiquitin antibodies, the presence or absence of degeneration of nerve cells, etc.
- Alzheimer's disease: the presence or absence of Aβ or tau accumulation, the presence or absence of neurofibrillary tangles, the presence or absence of reactivity by anti-ubiquitin antibodies, the presence or absence of degeneration of nerve cells, etc.
- Creutzfeldt-Jakob disease: the presence or absence of prion accumulation, the presence or absence of degeneration of nerve cells, etc.
- Huntington's disease: the presence or absence of huntingtin accumulation, the presence or absence of degeneration of nerve cells, etc.

When the neurodegenerative disease is Alzheimer's disease, i.e., when the target substance of screening is a therapeutic agent for Alzheimer's disease, nerve cells, into which Aβ or tau has been introduced, are preferably used as cells for the screening of candidate substances.

When the neurodegenerative diseases are Creutzfeldt-Jakob disease, Gerstmann-Straussler syndrome, and mad cow disease, it is preferable to use cells, into which prion proteins have been introduced. When the neurodegenerative diseases are Huntington's disease, spinal and bulbar muscular atrophy, spinocerebellar ataxia and dentatorubral-pallidoluysian, it is preferable to use nerve cells, into which polyglutamine has been introduced. When the neurodegenerative disease is amyotrophic lateral sclerosis, it is preferable to use nerve cells into which TDP-43 has been introduced. When the neurodegenerative diseases are FTDP-17, progressive supranuclear palsy, corticobasal degeneration and Pick disease, tau is preferably used.

Examples of the candidate substance may include a peptide, a protein, a non-peptidic compound, a synthetic compound (a high or low molecular weight compound), a fermentation product, a cell extract, a cell culture supernatant, a plant extract, tissue extracts from mammals (e.g., a mouse, a rat, a pig, a bovine, sheep, a monkey, a human, etc.), and a plasma. These compounds may be either novel compounds or known compounds. These candidate substances may form salts, and as such salts of candidate substances, salts with physiologically acceptable acids (e.g., inorganic acids, organic acids, etc.) or with bases (e.g., metallic acids) and the like may be used.

Cell death is induced to cells in which the seed activity-possessing variant protein or an aggregate thereof is accumulated. Thus, when a certain candidate substance is administered, if results from which alleviation or elimination of cell death can be confirmed can be obtained, the used candidate substance can be selected as a therapeutic agent for neurodegenerative disease.

The cells of the present invention can be provided in the form of a screening kit for a substance that suppresses intracellular accumulation of the neurodegenerative disease-associated protein or a therapeutic agent for neurodegenerative disease. The kit of the present invention includes the above-described cells, and may also include a labeling substance, a reagent for detection of cell death (e.g., LDH, etc.), and the like. The labeling substance means an enzyme, a radioisotope, a fluorescent compound, a chemiluminescent compound, etc. The kit of the present invention may include, in addition to the above-described components, other reagents for carrying out the method of the present invention, such as an enzyme substrate (chromogenic substrate, etc.), an enzyme substrate lysate, an enzyme reaction stopper solution, and the like, when the labeling is an enzyme labeling. Furthermore, the kit of the present invention may also include a diluent for test compounds, various types of buffers, sterile water, various types of cell culture vessels, various types of reaction vessels (e.g. Eppendorf tubes, etc.), detergents, experimental operating manuals (instructions), etc.

### Examples

Hereinafter, the present invention will be more specifically described in the following examples. However, these examples are not intended to limit the scope of the present invention.

### [Example 1] Alpha-synuclein variants

### < Experimental Methods >

### Preparation of recombinant alpha-synuclein (aS) monomer

The expression plasmid pRK172-aS (provided by Dr. Michel Goedert, Medical Research Council, Laboratory of Molecular Biology, U.K.), into which human wild-type or variant aS cDNA had been inserted, was transformed into *Escherichia coli* BL21/DE3 (Merck, Cat. #69450). The cells were cultured overnight at 37°C on an LB plate supplemented with 50 µg/mL sodium ampicillin (FUJIFILM Wako Pure Chemical Corporation, Cat. #012-23303). The cell mass was recovered, and was added to 500 mL of LB medium supplemented with sodium ampicillin to a final concentration of 50 µg/mL, followed by performing culture for 4 hours. Thereafter, isopropyl-β-thiogalactopyranoside (IPTG, FUJIFILM Wako Pure Chemical Corporation, Cat. Cat. #094-05144) was added to the cultured cells to a final concentration of 0.6 mM, and the obtained mixture was further cultured for 4 hours.

The culture medium was transferred into a centrifuge tube and was centrifuged at 4°C at 2,700 g for 8 minutes. The cell masses were recovered and was cryopreserved at -80°C. Thereafter, the cell mass was thawed, and was then suspended in 10 mL of buffer for aS purification (50 mM Tris-HCl, pH 7.5/1 mM EGTA/1 mM EDTA/1 mM DTT). The obtained suspension was transferred into a centrifuge tube and was then subjected to ultrasonic disintegration on ice for 1 minute. This suspension was centrifuged at 4°C at 26,600 g for 15 minutes, and the supernatant was then recovered. To the supernatant, 50 µL of 2-mercaptoethanol was added, and the obtained mixture was then subjected to a heat treatment at 100°C for 5 minutes. The reaction mixture was returned onto ice, and after slightly cooling the mixture, it was centrifuged at 4°C at 26,600 g for 15 minutes.

The supernatant was recovered, was then adsorbed on a Q Sepharose Fast Flow (Cytiva, Cat. #17051001) column (column volume: 2 mL) that had been equilibrated with a buffer for aS purification in advance, and was then washed with 20 mL of the aS purification buffer. Thereafter, the column was washed with 6 mL of aS purification buffer containing 0.1 M NaCl, and then, 6 mL of aS purification buffer containing 0.35 M NaCl was added to the column, so as to elute the protein adsorbed on the column. Ammonium sulfate was added to the eluate to reach 50% saturation, and the protein was then precipitated by leaving the eluate at rest on ice for 15 minutes. The precipitate was centrifuged at 4°C at 26,600 g for 15 minutes to remove the supernatant, and the obtained precipitate was dissolved in 30 mM Tris-HCl (pH 7.5).

The thus obtained solution was dialyzed overnight against the same buffer solution as described above, and was centrifuged at 4°C at 135,000 g for 20 minutes to remove insoluble matters, etc. Subsequently, the solution was analyzed by reversed-phase high-performance liquid chromatography using a Brownlee Aquapore RP-300 Column (30 mm × 4.6 mm I.D., PerkinElmer, Cat #07110055) (RP-HPLC: Agilent Technologies), so that the concentration of the protein in the solution was measured. This protein was defined to be a recombinant aS monomer.

### Preparation of aS aggregate

200 µL of the purified recombinant aS monomer solution (3-7 mg/mL) was placed in a 1.5-mL tube, and sodium azide was then added to the solution to a final concentration of 0.1%. This mixed solution was incubated for 1 week, while it was shaken at a rotational speed of 194 min⁻¹ using a shaker (TAITEC NR-3) equipped in a 37°C incubator. After one week, the monomer solution was solidified into a jelly-like state, and this was centrifuged at 135,000 g for 20 minutes to recover the aS aggregate as precipitate. To this precipitate, 200 µL of normal saline was added, and the obtained mixture was centrifuged in the same manner as that described above, so as to remove the remaining monomer.

The aS aggregate obtained as a precipitate was resuspended in 200 µL of normal saline, and was then subjected to an ultrasonic treatment (TAITEC CORPORATION, VP-050 and BRANSON, Sonifier SFX). A portion (5 µL) was aliquoted from the solution, and was mixed with 15 µL of 6 M guanidine hydrochloride, and thereafter, the protein concentration of aS fibers was measured using RP-HPLC.

### Preparation of variant aS

Variant aS was prepared using the QuickChange Site-Directed Mutagenesis Kit (Stratagene). In order to prepare a variant in which Lys43 or Lys45 is substituted with Ala (K43A or K45A) or Arg (K43R or K45R), a variant in which both Lys43 and Lys45 are substituted with Ala (K43&45A), Arg (K43&45R) or Glu (K43&45E), and a variant in which His50 is substituted with Ala (H50A), using pRK172-aS or pcDNA3-aS (a vector for expression of cultured cells) encoding wild-type aS as a template, and PCR was carried out with the following primers (Table 4).

### [Table 4]

**Table 4**

| Primer name | Sequence | |
|---|---|---|
| K43A-FW | GTGTTCTCTATGTAGGCTCCGCAACCAAGGAGGGAGTGGTGCA | 49 |
| K43A-RV | TGCACCACTCCCTCCTTGGTTGCGGAGCCTACATAGAGAACAC | 50 |
| K45A-FW | TCTATGTAGGCTCCAAAACCGCGGAGGGAGTGGTGCATGGTGT | 51 |
| K45A-RV | ACACCATGCACCACTCCCTCCGCGGTTTTGGAGCCTACATAGA | 52 |
| K43R-FW | GTGTTCTCTATGTAGGCTCCAGAACCAAGGAGGGAGTGGTGCA | 53 |
| K43R-RV | TGCACCACTCCCTCCTTGGTTCTGGAGCCTACATAGAGAACAC | 54 |
| K45R-FW | TCTATGTAGGCTCCAAAACCAGGGAGGGAGTGGTGCATGGTGT | 55 |
| K45R-RV | ACACCATGCACCACTCCCTCCCTGGTTTTGGAGCCTACATAGA | 56 |
| K43&45 A-FW | GAGGGTGTTCTCTATGTAGGCTCCGCAACCGCGGAGGGAGTGGTGCATGGT GTGGCA | 57 |
| K43&45 A-RV | TGCCACACCATGCACCACTCCCTCCGCGGTTGCGGAGCCTACATAGAGAAC ACCCTC | 58 |
| K43&45 R-FW | GAGGGTGTTCTCTATGTAGGCTCCCGAACCCGGGAGGGAGTGGTGCATGGT GTGGCA | 59 |
| K43&45 R-RV | TGCCACACCATGCACCACTCCCTCCCGGGTTCGGGAGCCTACATAGAGAAC ACCCTC | 60 |
| H50A-FW | TCCAAAACCAAGGAGGGAGTGGTGGCTGGTGTGGCAACAGTGGCTGAGA AG | 61 |
| H50A-RV | CTTCTCAGCCACTGTTGCCACACCAGCCACCACTCCCTCCTTGGTTTTGGA | 62 |
| K43&45 E-FW | GAGGGTGTTCTCTATGTAGGCTCCGAAACCGAGGAGGGAGTGGTGCATGGT GTGGCA | 63 |
| K43&45 E-RV | TGCCACACCATGCACCACTCCCTCCTCGGTTTCGGAGCCTACATAGAGAAC ACCCTC | 64 |

Moreover, a K43&45 delta variant, in which both Lys43 and Lys45 are deleted, was prepared employing KOD-Plus-Mutagenesis Kit (Toyobo Co., Ltd., Cat. #SMK-101) and using the following primers.
K43&45 delta-FW
   ACCGAGGGAGTGGTGCATGGTGTGGCAAC (SEQ ID No: 65)
K43&45 delta-RV
   GGAGCCTACATAGAGAACACCCTCTTTTGT (SEQ ID No: 66)

To the amplified PCR product (50 µL), 1 µL of DpnI (Toyobo Co., Ltd., Cat. #DPN-101) was added, and the obtained mixture was then incubated at 37°C for 1 hour to decomposed the plasmid added as a template. From this reaction solution, a portion (3 µL) was aliquoted, and was then mixed with 15 µL of *Escherichia coli* DH5 alpha competent cells (Toyobo Co., Ltd., Cat. #DNA-903F), and then, the thus obtained mixture was incubated at 42°C for 45 seconds for transformation. To this reaction solution, 100 µL of LB medium was added, followed by performing incubation at 37°C for 1 hour. Thereafter, the entire amount was seeded on an ampicillin-resistant LB plate, and the variant plasmids were obtained from the obtained colonies. The introduced mutations were confirmed by DNA sequencing analysis of the obtained plasmids.

### Measurement of fibrosis of aS monomers using thioflavin (Th)T

300 µL of wild-type or variant aS monomer (1 mg/mL) was placed in a 1.5-mL tube, and sodium azide was then added thereto to a final concentration of 0.1%. This was shaken at 37°C to induce the fibrosis of aS. On Days 0, 2, 5, 8, 12, and 16 after initiation of the fibrosis, 10 µL of sample was aliquoted and was then mixed with 300 µL of 20 mM Hepes (pH 7.5) containing 4 µM ThT (FUJIFILM Wako Pure Chemical Corporation, Cat. #202-01002). The obtained mixture was incubated at 37°C for 30 minutes. After the reaction, the sample (150 µL) was transferred to a 96-well plate, and the fluorescence intensity of ThT at an excitation wavelength of 442 nm and at a fluorescence wavelength of 485 nm was measured using a plate reader infinite M200 PRO (TECAN).

### Electron microscopic observation

3 µL of each aggregate (0.05 mg/mL) was added to a collodion membrane-adhered mesh (400 mesh, Nissin EM, Cat. #6512), and it was then left at rest at room temperature for 1 minute. Thereafter, water on the mesh was absorbed with a Kim's wipe, and 10 µL of 2% sodium phosphotungstate solution was then added to the mesh, followed by leaving at rest at room temperature for 3 minutes. Thereafter, the water on the mesh was absorbed with a Kim's wipe, and the mesh was left as was to dry naturally for several minutes. The dried mesh was subjected to JEM-1400 electron microscopy (JEOL) to observe the fiber structure of each aggregate.

### Measurement of seed activity of aS aggregate using ThT

100 µL of wild-type aS monomer (1 mg/mL), 10 µL of 1 M Hepes (pH 7.5), 10 µL of 400 µM ThT, and 5 µL of each aggregate (0.2 mg/mL) were mixed with one another in a 96-well plate. Using an infinite M200 PRO plate reader (TECAN), the fluorescence intensity of ThT in each sample was continuously measured at 37°C, at an excitation wavelength of 442 nm and at a fluorescence wavelength of 485 nm.

### Cultured cells

The human neuroblastoma cell line SH-SY5Y (American Type Culture Collection, Cat. #CRL-2266) purchased from the American Type Culture Collection was used. DMEM (Dulbecco's modified eagle's medium nutrient mixture)/F-12HAM (Sigma-Aldrich, Cat. #D8062-500ML), to which 10% (v/v) fetal bovine serum, a non-essential amino acids solution (MEM Non-Essential Amino Acids Solution (100X), ThermoFisher, Cat. #11140050), and a penicillin-streptomycin-glutamine solution (Penicillin-Streptomycin-Glutamine (100X), ThermoFisher, Cat. #10378016) had been added, was used as a culture medium, and the cells were cultured at 37°C in a 5% CO₂ incubator (Thermo SCIENTIFIC). For the culture, a collagen-coated 6-cm petri dish (BD Biocoat) and a 6-well plate (BD Biocoat) were used.

Cell passaging was performed according to the following procedures, in a state in which the cells became 100% confluent. After removing the medium from the 6-cm petri dish, the cells were then washed with 1.5 mL of normal saline, and the normal saline was then removed. Thereafter, 1 mL of 0.25% trypsin was added thereto, and the cells were incubated at 37°C for 5 minutes. Thereafter, 2 mL of fresh medium was further added, and after the trypsin reaction was terminated, the cells were fully suspended and were then seeded on a 6-cm petri dish, to which 3 mL of medium had been added. In general, when 4 to 6 × 10⁵ cells are added to a 6-cm petri dish containing 3 mL of medium, the cells become almost 100% confluent (2 to 3 × 10⁶ cells/mL) in about 3 days.

### Expression of aS plasmid and introduction of recombinant aS aggregate into cells

Regarding the seed activity of an aS aggregate in cultured cells, the method (3) of Nonaka et al. was applied.

In a 6-well plate, 8 ×10⁵ cells per well were seeded and were then cultured overnight. The next day, a wild-type aS expression plasmid (pcDNA3-aS) was introduced into the cells using X-treamGENE9 (Roche, Cat. #6365809001). Specifically, Opti-MEM (ThermoFisher, Cat. #31985062), the plasmid, and X-treamGENE9 were gently mixed with one another at a ratio of 100 µL : 1 µg : 3 µL, and the mixture was then left at rest at room temperature for 15 minutes. Thereafter, the mixed solution was added dropwise into the culture medium in each well. After 3 to 5 hours, 1.2 µL of recombinant aS aggregate (0.2 mg/mL) was added to the culture medium in each well. The treated cells were incubated in a CO₂ incubator, and 2 days later, the cells were recovered as follows.

### Detection of insoluble aS by immunoblotting method

The culture medium was removed from each well using an aspirator, and 1 mL of normal saline was then added thereto, so that the cells were recovered by peeling them from the plate. The cells were recovered by centrifugation at 1,800 g for 5 minutes, and A68 buffer (10 mM Tris-HCl (pH 7.5)/1 mM EGTA/10% sucrose/0.8 M NaCl) containing 300 µL of 1% sarkosyl (N-lauroyl sarcosine sodium salt, Sigma-Aldrich, Cat. #L5125-500G) was added to the cells. The obtained mixture was treated with a TAITEC VP-050 ultrasonic treatment device (intensity: PWM 17%) for 40 to 60 seconds, so as to disintegrate the cells. Thereafter, 300 µL of A68 buffer containing 1% sarkosyl was further added, and the cells were centrifuged at 113,000 g for 20 minutes (himac CS100GXL, Eppendorf Himac Technologies, Co., Ltd.).

300 µL of the obtained supernatant (sarkosyl supernatant fraction: Sar-sup) was recovered, and then, 75 µL of 5x SDS sample buffer (5 × SB) containing 5% 2-mercaptoethanol was added thereto. Moreover, using 15 µL of the supernatant, the amount of a protein in the supernatant fraction was quantified by BCA assay (BCA Protein Assay Kit, ThermoFisher, Cat. #23225). On the other hand, after addition of 5 × SB containing 50 µL of 5% 2-mercaptoethanol, the precipitate fraction was subjected to an ultrasonic treatment and was heat-treated at 100°C for 5 minutes, so that the resultant was recovered as a sarkosyl-insoluble fraction (Sar-ppt).

The obtained samples (Sar-sup and Sar-ppt) were electrophoresed on 13.5% polyacrylamide gels, and were then transcribed onto PVDF membranes (Millipore) under conditions of 200 mA for 1 hour. The PVDF membranes were blocked with a normal saline containing 3% gelatin (FUJIFILM Wako Pure Chemical Corporation, Cat. #077-03155) at room temperature for 10 minutes, and were then allowed to react at room temperature overnight with primary antibodies diluted with a normal saline containing 0.1% NaN₃ (10% CS/normal saline) (an antibody recognizing phosphorylated aS (phosphorylation of 129th residue, Ser) (anti-phosphorylated a-synuclein monoclonal antibody (pSyn#64) : anti-64 antibody, 1 : 1,000 dilution, FUJIFILM Wako Pure Chemical Corporation, Cat. #015-25191), and an antibody recognizing the C-terminus of aS (131-140 residues) (anti-a-synuclein (131-140) : anti-131-140, 1 : 1,000 dilution, COSMO BIO CO., LTD., Cat. #TIP-SN-P09).

Thereafter, the PVDF membranes were washed with several mL of normal saline, and were then allowed to react with a secondary antibody diluted with 10% CS/normal saline (1 : 1,000 dilution, Biotin-Goat anti mouse IgG, Vector, Cat. #BA-9200-1.5 or Biotin- Goat anti rabbit IgG, Vector, Cat. #BA-1000-1.5) at room temperature for 1 hour, followed by washing with Tris saline (TS). The PDVF membranes were allowed to react with a peroxidase-labeled avidin-biotin complex (ABC Standard Kit, Vector, Cat. #PK-4000) for 30 minutes, were then washed with a normal saline, and were then treated with a normal saline containing 0.1% 3,3'-Diaminobenzidine (Sigma-Aldrich, Cat. #D8001-5G), 0.2 mg/mL nickel(II) chloride hexahydrate (FUJIFILM Wako Pure Chemical Corporation, Cat. #141-01045) and 0.05% H₂O₂ (hydrogen peroxide water, Sigma-Aldrich Japan, Cat. #13-1910-5), so that protein bands on the membranes were chromogenized. The coloration reaction was terminated by washing the PVDF membranes with tap water.

### Mice

Male wild-type mice (C57BL/6J) were purchased from Japan SLC, Inc., and were bred in the animal breeding facility of the Tokyo Metropolitan Institute of Medical Science.

### Samples to be inoculated into mouse brain

The samples to be inoculated into mouse brain are as follows.
(1) Normal saline (5 µL): used as a negative control.
(2) 10 µg of human wild-type aS fibers (2 mg/mL, 5 µL)
(3) 10 µg of variant K43&45A fibers (2 mg/mL, 5 µL)
(4) 10 µg of variant K43&45R fibers (2 mg/mL, 5 µL)

### Method of inoculating samples into mouse brain

Mice were placed in an anesthesia bottle containing a tissue paper moistened with an anesthetic (20% isoflurane, Japanese Pharmacopoeia, Pfizer), and were then left at rest for several minutes. Thereafter, the anesthetized mice were immobilized with an auxiliary ear bar and were further anesthetized by inhalation of isoflurane. A recombinant aS aggregate was inoculated as a sample into the striatum of the right brain of mice as follows. First, the scalp of each mouse was incised to confirm the location of the bregma, a distance of 2 mm horizontally and 0.5 mm vertically from the location of the bregma was then marked, and a hole was then drilled through the skull at the marked position. A syringe (HAMILTON, Cat. #80301) containing the sample was inserted to a depth of 3 mm, while preventing the needle from bending, and 5 µL of the sample was inoculated. In order to reduce the leakage of the sample, the syringe was left at rest for 1 minute, and the needle was then removed, followed by suturing. Finally, for identification purposes, the ears were punched, and the mice were then returned to the cage.

### Excision of mouse brain

Three months after inoculation of the mouse brains with the samples, the brains were excised. The mice were anesthetized by inoculating 0.1 mL of somnopentyl (Schering-Plough) into the abdominal cavity, and the blood was removed from the whole body using a normal saline (Terumo Normal Saline, type B, Terumo), and the brain was then removed. The brains were carefully removed using surgical scissors and tweezers in order to avoid tissue damage.

### Immobilization of mouse brain

The excised mouse brains were immobilized at 4°C in 10% neutral buffered formalin solution (FUJIFILM Wako Pure Chemical Corporation, Cat. #062-01661). Since the brain tissues are not only disintegrated over time, but also an antigen of interest is likely to leak out, the tissues were immobilized as quickly as possible (for 3-4 days).

### Preparation of vibratome section and immunohistochemical analysis

Using a vibratome device (Leica) equipped with a feather steel double-blade (blue blade), the immobilized mouse brain was sliced into 30 µm-thick slices, so as to prepare sections. The sections were attached to anti-exfoliation coated glass slides (MATSUNAMI), and were then dried. Subsequently, the sections were autoclaved in a 0.01 M sodium citrate buffer at 105°C for 10 minutes. Thereafter, the sections were reacted in 100% formic acid for 10 minutes, were then rinsed with running water, and were then immersed in methanol containing 3% H₂O₂.

Thereafter, the sections were washed three times with a normal saline (Tx/normal saline) containing 0.03% TritonX-100, and were blocked with 10% CS for 20 minutes. Subsequently, the sections were reacted with a 1,000-fold diluted phosphorylated aS antibody (anti-pS129, abcam, Cat. #ab51253) overnight. The next day, the resulting sections were washed three times with Tx/normal saline, and were then reacted with a 500-fold diluted biotinylated anti-mouse IgG (H + L) antibody at room temperature for 1.5 hours. Thereafter, the sections were washed three times with Tx/normal saline, and were then reacted with an avidin-biotin-peroxidase complex using ABC Kit at room temperature for 1 hour. The sections were washed three times with Tx/normal saline, and were then chromogenized with a chromogenic solution prepared at the time of use (TS containing 1% DAB and 1% H₂O₂). The coloration reaction was terminated by washing the glass slides with tap water.

After drying, these sections were treated with Mayer's Hematoxylin (MUTO PURE CHEMICALS CO., LTD., Cat. #30002) for 1 minute for nuclear staining, and were then washed with running water for 5 minutes. The specimens were completely dried, were immersed in xylene for 10 minutes, and were then dehydrated, and thereafter, the specimens were encapsulated in Antifade Mounting Medium for fluorescence (VECTASHIELD, Cat. #H-1000-10). The thus obtained preparations were stored at room temperature. An all-in-one microscope (KEYENCE: BZ-X710) was used for observation.

### Vaccine therapy using variant K43&45A aggregate

According to the scheme shown in Figure 10, mouse brains that had previously been immunized with a K43&45aggregate as a vaccine were inoculated with an aS aggregate, so as to examine whether the seed-dependent formation of the aS aggregate in the brain is suppressed by the vaccine treatment.

### Administration of immunogen

Six mice (6-8 weeks old) were divided into three groups of two mice each. The mice were placed in an anesthesia bottle containing a tissue paper moistened with an anesthetic (20% isoflurane: Pfizer), and were then left at rest for several minutes. The backs of the anesthetized mice were subcutaneously inoculated with the following samples at several locations.

### (1) Group immunized with normal saline

125 µL of normal saline (Otsuka Normal Saline Injection, Otsuka Pharmaceutical Co., Ltd.) and 125 µL of adjuvant (Freund's complete adjuvant was used only for the first inoculation, and then, Freund's incomplete Use Freund's complete adjuvant for the second or third inoculation: Freund's complete adjuvant, Sigma-Aldrich, Cat. #F5881-10ML; Freund's incomplete adjuvant, Sigma-Aldrich, Cat. #F5506-10ML) were mixed with each other in a 1.5-mL tube and were then subjected to an ultrasonic treatment using an ultrasonic treatment device (TAITEC CORPORATION, VP-050) until a white emulsion was formed. For each immunization, the whole amount of emulsion was subcutaneously inoculated into the backs of one mouse at several locations.

### (2) Group immunized with 20 µg of K43&45A aggregate (vaccine)

20 µg of a K43&45A aggregate was mixed with a normal saline (total amount: 125 µL), and 125 µL of adjuvant (Freund's complete adjuvant was used only for the first inoculation, and then, Freund's incomplete Use Freund's complete adjuvant for the second or third inoculation) was further added thereto. The obtained mixture was subjected to an ultrasonic treatment using an ultrasonic treatment device (TAITEC CORPORATION, VP-050) until a white emulsion was formed. For each immunization, the whole amount of emulsion was subcutaneously inoculated into the backs of one mouse at several locations.

### (3) Group immunized with 50 µg of K43&45A aggregate

50 µg of a K43&45A aggregate was mixed with a normal saline (total amount: 125 µL), and 125 µL of adjuvant (Freund's complete adjuvant was used only for the first inoculation, and then, Freund's incomplete Use Freund's complete adjuvant for the second or third inoculation) was further added thereto. The obtained mixture was subjected to an ultrasonic treatment using an ultrasonic treatment device (TAITEC CORPORATION, VP-050) until a white emulsion was formed. For each immunization, the whole amount of emulsion was subcutaneously inoculated into the backs of one mouse at several locations.

### (4) Group immunized with 100 µg of K43&45A aggregate

100 µg of a K43&45A aggregate was mixed with a normal saline (total amount: 125 µL), and 125 µL of adjuvant (Freund's complete adjuvant was used only for the first inoculation, and then, Freund's incomplete Use Freund's complete adjuvant for the second or third inoculation) was further added thereto. The obtained mixture was subjected to an ultrasonic treatment using an ultrasonic treatment device (TAITEC CORPORATION, VP-050) until a white emulsion was formed. For each immunization, the whole amount of emulsion was subcutaneously inoculated into the backs of one mouse at several locations.

### Measurement of antibody titer in peripheral blood of mice by ELISA (Enzyme-Linked Immunosorbent Assay)

In order to examine whether an antibody of interest is produced in mice by administration of a K43&45A aggregate (a vaccine), peripheral blood was collected from the tail of the mice, and an antibody titer was then measured by ELISA. Blood (50 to 200 µL) was collected from the tail vein of the mice at the time of the first and third administration of the vaccine. The collected blood was incubated at 37°C for 1 hour, and was then left at rest at 4°C overnight. Thereafter, serum was recovered by performing centrifugation at 10,000 g for 10 minutes, and was then preserved at -20°C until use. To each well of a 96-well plate (SUMILON: high adsorption and flat bottom), 50 µL of 2 ng/µL antigen solution (wild-type or K43&45A aggregate) was added, and it was then left at rest at 4°C overnight.

The antigen solution was removed from each well, and the wells were then washed twice with 100 µL of normal saline. To each well, 150 µL of 10% bovine serum (CS) was added, and it was then left at rest at room temperature for 90 minutes. The wells were washed twice with 200 µL of normal saline, and a serum solution (100 µL) that had been 500-fold diluted with 10% CS was added to the wells and was then left at rest at room temperature for 1 hour. Thereafter, the wells were washed three times with 150 µL of normal saline, and 100 µL of peroxidase-labeled goat anti-mouse IgG (BioRad, Cat. #170-6516) that had been 3,000-fold diluted with 10% CS was added to the wells, and was then left at rest at room temperature for 1 hour at room temperature. The wells were washed three times with 150 µL of citrate-phosphate buffer (pH 5.0), and thereafter, 100 µL of substrate solution (a solution prepared by mixing 6 mg of o-phenylenediamine (FUJIFILM Wako Pure Chemical Corporation, Cat. #160-11022), 10 mL of citrate-phosphate buffer (pH 5.0) and 10 µL of H₂O₂ with one another) was added thereto. The solution was left at rest at room temperature for 5-10 minutes to develop color.

After an appropriate time had passed, 20 µL of 2 N sulfuric acid was added to each well to terminate the coloration. The absorbance of each well at 490 nm was measured using a plate reader infinite M200 PRO (TECAN).

Inoculation of wild-type aS aggregate and detergent-insoluble fraction prepared from MSA patient brain into mouse brains administered with K43&45A aggregate (vaccine)

Two weeks after the first vaccine administration, the second administration was carried out, and further, two weeks after the second administration, the third administration was carried out. Two weeks after a total of the three vaccine administrations, 2.5 µg of recombinant mouse wild-type aS aggregate (5 µL at 0.5 mg/mL) was inoculated into the mouse brain (right brain and striatum).

The detergent-insoluble fractions (seed fractions) were prepared from MSA patient brain as follows. A frozen specimen (0.5 g) of the patient brain was homogenized in a 5-fold volume of A68 buffer (10 mM Tris, pH 7.5, 0.8M NaCl, 1 mM EGTS, and 1 mM DTT). To the obtained suspension, sarkosyl was added to a final concentration of 1%, and the obtained mixture was then stirred. The reaction mixture was incubated at 37°C for 30 minutes. Thereafter, this suspension was centrifuged (12,000 g, 10 minutes, 25°C), and the supernatant was then recovered. The supernatant was further centrifuged (113,000 g, 20 minutes, 25°C), and the precipitate was then recovered. An appropriate amount of normal saline was added to this precipitate, and the mixture was subjected to an ultrasonic treatment, and was then divided into 1.5-mL Eppendorf tubes in appropriate amounts. After that, each aliquot was centrifuged (113,000 g, 20 minutes, 25°C), and the precipitate (insoluble fraction of the patient brain) was recovered. To one of the insoluble fractions, 50 µL of normal saline was added, and it was then subjected to an ultrasonic treatment (TAITEC CORPORATION, VP-050). An aliquot (5 µL) of this suspension was inoculated as a seed into the mouse brain (right brain, striatum).

### Immunohistochemical analysis of mouse brain

One month after inoculation with the aggregate, mouse brains were excised and immobilized, and vibratome sections were then prepared. The sections were stained with a phosphorylated aS-specific antibody (anti-pS129, abcam, Cat. #ab51253), and were then observed using an all-in-one microscope (KEYENCE: BZ-X710). At the same time, the area value of the anti-pS 129-positive structure (phosphorylated aS aggregate) that appeared in the mouse brain was calculated and was defined as an aggregate amount.

### Preparation of variant aS aggregate by addition of wild-type aS aggregate

A wild-type aS monomer (6.1 mg/mL, 200 µL) and a K43&45A monomer (7.2 mg/mL, 200 µL), a K43&45R monomer (6.3 mg/mL, 200 µL) or a K43&45 delta monomer (6.7 mg/mL, 200 µL) were mixed with a wild-type aS aggregate (1 µg), and sodium azide was added to the mixture to reach a final concentration of 0.1%. The thus obtained mixture was incubated at 37°C for 12 days. Those samples were centrifuged at 135,000 g at 25°C for 20 minutes, and the aS aggregate was recovered as a precipitate. To this, 200 µL of normal saline was added, and the obtained mixture was centrifuged in the same manner as described above, so as to remove the remaining monomers. The aS aggregate obtained as a precipitate was resuspended in 200 µL of normal saline, and was then subjected to an ultrasonic treatment (TAITEC CORPORATION, VP-050, and BRANSON, Sonifier SFX). An aliquot (5 µL) of this resultant was mixed with 15 µL of 6 M guanidine hydrochloride, and the protein concentration of the aS aggregate was then measured by RP-HPLC.

### < Results >

### 1. Novel vaccine therapy using variant aS aggregate

From the three-dimensional structures of aS aggregates derived from MSA patient brain and recombinant aggregates, which were observed by cryo-electron microscopy and were published in 2020 (Figure 1), it was elucidated the three-dimensional structure is based on a core structure consisting of two molecules of aS protofilaments (PF: a precursor of amyloid fibril) (1, 2). Since basic amino acids such as Lys43, Lys45, or His50 are concentrated in the central portion of the interaction of the two molecules of PF, the present inventors have assumed that these amino acids may be important for the polymerization of aS. Thus, we have focused their attention on Lys43 and Lys45, have produced variants in which amino acid substitution mutations were introduced into this portion, and have then examined their properties. The produced variants were a K43&45A variant in which Lys43 and Lys45 are both substituted with Ala, a K43&45R variant in which they are both substituted with Arg, a K43&45E variant in which they are both substituted with Glu, a K43&45 delta in which K43 and K45 are both deleted, and the like.

These recombinant wild-type and variant (K43&45A and K43&45R) monomers were prepared, and were observed for their *in vitro* aggregate formation (Figure 2). A 1 mg/mL monomer solution was shaken at 37°C, and was allowed to react with thioflavin T (ThT) after a certain period of time had passed, so that the fluorescence intensity was measured. ThT does not react with monomer proteins, but it reacts with protein aggregates having a β sheet structure-rich amyloid fiber structure, and emits fluorescence. As a result, it was clarified that an increase in the fluorescence intensity of ThT was observed over time in both of the two types of prepared variants, and that they were aggregated when shaken at 37°C as in the case of the wild-type aS. When these aggregates were observed by electron microscopy, fibrous structures were observed in all samples (Figure 3). In other words, it became clear that the variant aS aggregate prepared this time has a fibrous structure similar to that of the wild-type aggregate.

The aS monomers are aggregated when shaken at 37°C, but are not aggregated when left at rest without shaking. However, when a small amount of aS fibers prepared in advance is added to the monomer solution that is left at rest, the monomers are aggregated. In other words, it is considered that the added aS aggregate acts as an aggregation seed (seed), and the aS monomers are aggregated in a seed-dependent manner. Subsequently, for the purpose of examining the seed activity of these aggregates, such an aggregate (1 µL) was added to a wild-type monomer solution (1 mg/mL, 100 µL), and the fluorescence intensity of ThT was then measured (Figure 4). The wild-type aS monomer (none: no seed) without addition of the aggregate did not increase the fluorescence intensity of ThT at all, even after incubation at 37°C.

When the wild-type aggregate or the K43&45R aggregate was added as a seed to the wild-type aS monomer, an increase in the fluorescence intensity of ThT over time was observed in both cases, and thus, it was elucidated that these aggregates had seeding effects on the wild-type aS monomer. It became clear that, in particular, the seeding effects of the K43&45R aggregate were extremely high, and were about four times higher than those of the wild-type aggregate. On the other hand, even when the K43&45A aggregate was added to the wild-type aS monomer, the fluorescence intensity of ThT did not increase over time at all. That is to say, these results suggest that this variant K43&45A aggregate has almost no seeding effects.

Next, K43 and K45 variants, into one residue each of which a mutation was introduced, were analyzed in the same manner as described above. Specifically, K43R, K45R, K43A and K45A variant monomers were prepared, and were shaken at 37°C for 1 week to obtain the respective aggregates. For the purpose of examining the seed activity of these aggregates, the aggregates (1 µg) were each added to a wild-type monomer solution (1 mg/mL, 100 µL), and the fluorescence intensity of ThT was then measured (Figures 5 and 6). As a result, in the case of variant aggregates substituted with Arg, as shown in Figure 5, the strongest seed activity was observed in the K45R aggregate, followed by the K43&45R aggregate, K43R aggregate and wild-type aggregate in this order. On the other hand, the seeding effects of the Ala-substituted variant aggregates were all lower than those of the wild-type aggregate, and the order was the wild-type aggregate > the K43A aggregate > the K45A aggregate > the K43&45A aggregate (Figure 6).

The variant in which both K43 and K45 were substituted with Glu (K43&45E) and the variant in which both of the amino acids were deleted (K43&45 delta) were also analyzed in the same manner as described above. Monomers of K43&45E and K43&45 delta variants were prepared, and these monomers were shaken at 37°C for 1 week to obtain the respective aggregates. For the purpose of examining the seed activity of these aggregates, the aggregates (1 µg) were each added to a wild-type monomer solution (1 mg/mL, 100 µL), and the fluorescence intensity of ThT was then measured ( Figure 7). As a result, it was revealed that the seed activity of both K43&45E and K43&45 delta aggregates was much lower than that of the wild-type aggregate, and that, similarly to the previously shown K43&45A aggregate, both of the K43&45E and K43&45 delta aggregates had almost no seeding effects.

In the vicinity of K43 and K45, an amino acid similarly having a positive charge, H50, is present. Next, the influence of H50 on the seeding effects of an alpha-synuclein aggregate was examined. An H50A variant monomer was prepared by substituting H50 with Ala, and was then shaken at 37°C for 1 week to obtain an H50A aggregate. For the purpose of examining the seed activity of this aggregate, the H50A aggregate (1 µg) was added to a wild-type monomer solution (1 mg/mL, 100 µL), and the fluorescence intensity of ThT was then measured ( Figure 8). As a result, the seed activity of the H50A aggregate was lower than that of the wild-type aggregate, but higher than that of the K43&45A aggregate.

The seeding effects of recombinant aS aggregates were examined using cultured cells. When an aS expression plasmid is allowed to transiently express in the cultured cells SH-SY5Y, aS derived from the plasmid is mainly expressed as a soluble protein in the cytoplasm and is hardly insoluble. It has been reported that when a previously prepared aS aggregate is added to the aS plasmid-expressing cells in a medium, the aS aggregate is incorporated into the cells and acts as a seed in the cytoplasm, resulting in intracellular accumulation of soluble aS derived from the plasmid (3). Thus, using this method, the seeding effects of two types of variant aggregates were examined using cultured cells.

0.24 µg of each different type of aggregate was introduced as a seed into SH-SY5Y cells transiently expressing wild-type aS. After performing culture for 2 days, the cells were recovered and were then homogenized in a buffer containing one type of detergent, sarkosyl. The homogenate was subjected to ultracentrifugation to obtain a supernatant (sarkosyl-soluble fraction: Sar-sup) and a precipitate (sarkosyl-insoluble fraction: Sar-ppt). These fractions were subjected to immunoblotting using anti-131-140 (an antibody recognizing the C-terminus of aS) and anti-64 (a phosphorylated aS-specific antibody), and the obtained anti-64 positive bands were quantified (Figure 9).

In aS accumulated in the patient brain, the 129th residue Ser is phosphorylated, but the soluble aS is hardly phosphorylated (4). Phosphorylation of aS is considered to be a post-translational modification that occurs after aggregation, and is a good marker for sensitive detection of intracellular aS aggregation. When immunoblotting is performed using anti-64, a few bands were detected in the soluble fraction of cells expressing only the plasmid, while almost no bands were observed in the insoluble fraction. In other words, most of the plasmid-derived aS is soluble in cultured cells and is not phosphorylated. However, when a wild-type aggregate was introduced as a seed into the cells, multiple bands positive for a phosphorylated aS-specific antibody were strongly detected in the insoluble fraction.

It is considered that the band with the smallest molecular weight (about 15 kDa) is the phosphorylated aS monomer, the band with about 25 kDa is aS to which one ubiquitin binds, the band with around 30 kDa is an aS dimer, the band with around 45 kDa is aS to which two ubiquitins bind, or an aS trimer. These multiple bands were also observed in the immunoblotting of aS accumulated in patient brains using a phosphorylated aS-specific antibody (5, 6), and it is easy to reproduce abnormal aS generated in patient brains in cultured cells. On the other hand, when two types of variant aggregates were introduced as seeds into cells, an anti-64 antibody-positive band was strongly detected in the case of using the K43&45R aggregate, while the phosphorylated aS band was hardly detected when the K43&45A aggregate was introduced. From these results, it became clear that the K43&45R variant aggregate functions as a seed in cells, as well as the wild-type aggregate, but the K434&45A aggregate hardly functions as a seed.

The seeding effects of aS aggregates were analyzed *in vivo.* When a recombinant aS aggregate is inoculated into wild-type mouse brain, it functions as a seed in the brain, and endogenous mouse aS is intracellularly accumulated in a seed-dependent manner. In other words, it is possible to reproduce the aS aggregate found in the patient brain in the mouse brain (6). The variant aS aggregate prepared in this study was inoculated into wild-type mouse brain in the same manner as described above, and the seed activity thereof was examined *in vivo.* After inoculation of a WT aggregate into mouse brain, endogenous mouse aS is accumulated in a seed-dependent manner in about 3 months, and a large number of phosphorylated aS aggregates appeared in the brain. Similarly, when a K43&45R aggregate was inoculated, phosphorylated aS aggregates in the brain were observed about 6 times larger than those in the brain inoculated with the WT aggregate (Figure 10). On the other hand, phosphorylated aS aggregates hardly appeared in the mouse brain inoculated with a K43&45A aggregate. These results demonstrate that the K43&45R aggregate has stronger seeding effects than the WT aggregate *in vivo,* as well as the results of *in vitro* as shown in Figure 4, whereas the K43&45A aggregate has almost no seeding effects.

Thus, variant aS that forms aggregates but has no seed activity (prion-like activity) has not been reported at all. We focused on the fact that the K43&45A aggregate has no seed activity and conceived that this aggregate could be used as a vaccine. This is because "almost no seed activity" is considered to be an "aggregate with less pathogenicity (i.e., an attenuated aggregate)" for living organisms, unlike the wild-type aggregate.

At present, three vaccine preparations have been reported as aS vaccine therapies from overseas pharmaceutical groups, all of which are based on aS monomers or peptides thereof modified to increase immunogenicity. From the viewpoint of vaccine, it is considered desirable to use aggregates, which are considered to have higher immunogenicity than monomers (because aS monomers exist in the body of healthy individuals, but aggregates do not exist therein).

However, the wild-type aS aggregate has seed activity (prion-like activity), and when it is inoculated into mouse brain, it exhibits prion-like activity and causes intracellular accumulation of mouse endogenous aS in a seed-dependent manner. That is, since the wild-type aS aggregate has pathogenicity for living organisms, its safety for living organisms cannot be completely guaranteed even if it is inoculated into the periphery as a vaccine. In general, in immunization against measles and other diseases, a part of bacteria or viruses that have been weakened and/or detoxified is inoculated as a vaccine into healthy individuals, so as to promote production of antibodies against them and to ultimately prevent the individuals from contracting the disease. Considering in the same way, the K43&45A aggregate is a weakened aggregate, and its utilization as a vaccine may prevent neurodegenerative disease associated with aS accumulation.

Thus, we examined whether or not the K43&45A aggregate functions as a vaccine (Figure 11). The K43&45A aggregate (20 to 100 µg) was mixed with a Freund's complete adjuvant (only for the first immunization) or a Freund's incomplete adjuvant (for the second immunization and thereafter), and the mixture was then subjected to an ultrasonic treatment to prepare an emulsion. The emulsion was subcutaneously inoculated into the skin of 6- to 8-week-old mice (a total of 3 times; with intervals of every 2 weeks). Before the immunization and after the three times of immunization, blood was collected, and whether or not an antibody of interest was produced was confirmed by ELISA. As a result, it was revealed that the mice vaccinated with 20 mg and 100 mg of the K43&45A aggregate as a vaccine all produced antibodies against the K43&45A aggregate inoculated as an immunogen (Figure 12). On the other hand, no such antibodies were produced at all in the group immunized with a normal saline.

Mouse brains that had been immunized a total of three times were inoculated with a mouse wild-type aS aggregate (2.5 µg) or a detergent-insoluble fraction (a fraction containing an aS aggregate) prepared from the brains of patients with multiple system atrophy (MSA) as a seed, and 1 month after the inoculation, the brains were excised. As a control, mouse brains that had been immunized with a normal saline, instead of a variant aggregate, were also inoculated with each seed, and the brains were then excised in the same manner as described above. After the excised brains had been fixed with 4% paraformaldehyde, the amount of seed-dependent endogenous mouse aS accumulated was analyzed by immunohistochemical analysis using a phosphorylated aS-specific antibody.

The results of the groups, in which a mouse wild-type aS aggregate was inoculated into the immunized mouse brains, are shown in Figure 13. In the group immunized with a normal saline, a large number of seed-dependent phosphorylated aS aggregates were observed in the mouse brain, while the aggregate formation was significantly suppressed in the group immunized with the K43&45A aggregate. Likewise, even when insoluble aS derived from MSA patient brain was inoculated into the immunized mice, a large number of phosphorylated aS aggregates were observed in the brain in the group immunized with a normal saline, but in the group previously immunized with the K43&45A aggregate, the aggregate formation was significantly suppressed (Figure 14).

These results demonstrated that the K43&45A aggregate functions as a vaccine in *in vivo* mouse models. In addition, it is also considered that aS variant-derived aggregates that exhibit almost no seed activity as with the K43&45A aggregate, such as K43&45E, K43&45 delta and H50A aggregates, may also function as a vaccine.

### 2. Suppression of aS aggregate formation by aS variant expression or delivery of aS variant into the brain using adeno-associated virus (AAV)

As mentioned above, it became clear that the K43&45A, K43&45E and K43&45 delta aggregates exhibit almost no seed activity, suggesting that these aggregates have lower pathogenicity than wild-type aggregates. From these results, the possibility that these variant aggregates can be used as vaccines against alpha-synucleinopathy was investigated, and their usefulness could be demonstrated. These variant aggregates can be prepared by shaking each monomer solution at 37°C (aggregates obtained by shaking), while the aS monomer has the property of seed-dependent aggregation under static conditions without shaking ( seed-dependent aggregate).

Thus, whether or not these variant aS monomers are aggregated in a seed-dependent manner was examined. Specifically, whether the variant aS monomers are aggregated when the wild-type aS aggregate (FWT) obtained by shaking is added as a seed to the variant aS monomer solution was examined. Wild-type aS and variant monomer solution (1 mg/mL, 100 µL) were mixed with the wild-type aS aggregate (FWT: 1 µg each) obtained by shaking, and the obtained mixture was then left at rest at 37°C for incubation. As a result, as shown in Figure 15, an increase in the fluorescence intensity of ThT over time was observed in all of the wild-type and three types of variant monomers, when FWT was added as a seed..

On the other hand, no increase in the fluorescence intensity of ThT was observed when no seed was added. Therefore, it was revealed that not only the wild-type aS monomer but also the K43&45A, K43&45R and K43&45 delta monomers are aggregated in a seed-dependent manner in the presence of the wild-type aS aggregate by shaking.

As shown in Figure 4, the seed activity of the variant K43&45A aggregate by shaking was hardly observed, but whether or not the seed-dependently aggregated variant aggregate prepared in Figure 15 has seed activity was examined. First, seed-dependent aggregates were prepared from wild-type and variant monomers, respectively, as follows. A wild-type aS (WT) monomer (6.1 mg/mL, 200 µL) and a K43&45A (KA) monomer (7.2 mg/mL, 200 µL), a K43&45R (KR) monomer (6.3 mg/mL, 200 µL), or a K43&45 delta (K delta) monomer (6.7 mg/mL, 200 µL) were mixed with the wild-type aS aggregate (FWT: 1 µg) by shaking, and the obtained mixture was left at rest at 37°C for 12 days for incubation.

Thereafter, these samples were centrifuged to recover individual seed-dependent aggregates (WT + FWT, KA + FWT, KR + FWT, and K delta + FWT). Next, the seeding activities of these aggregates were examined by comparing with one another. These aggregates were each added to the wild-type aS monomer, and the fluorescence intensity of ThT was then measured over time. As shown in Figure 16, FWT (an aggregate by shaking) and seed-dependent aggregates (WT + FWT, KR + FWT, K delta + FWT, and KA + FWT) were added to the WT monomer, and the obtained mixture was then left at rest at 37°C for incubation. Thereafter, the fluorescence intensity was measured over time. As a result, the seed activity in the case of adding the seed-dependent aggregates such as WT + FWT and KR + FWT was slightly lower than that in the case of adding FWT, but the seeding effects in the case of adding the seed-dependent aggregates such as K delta + FWT and KA + FWT were significantly lower than that of others.

From these results, it became clear that the seed-dependent aggregate obtained by adding FWT to the K43&45A monomer (KA + FWT) and the seed-dependent aggregate obtained by adding FWT to the K43&45 delta monomer (K delta + FWT) had considerably lower seeding effects. These results demonstrated that the expression of the K43&45A monomer or the K43&45 delta monomer in the brain suppresses aS aggregate formation (Figure 17). That is to say, it is considered that wild-type (endogenous) aS monomers are constantly expressed in the human brain, but when aS aggregates appear in the brain for some reason, they function as seeds and endogenous aS monomers are aggregated in a seed-dependent manner, and further, the aggregate functions as a seed to promote further aggregation of endogenous aS monomers, ultimately leading to the development of alpha-synucleinopathy (Figure 17-A).

Herein, if the K43&45A (KA) monomer can be expressed or delivered into the brain, a mixture of endogenous aS and the KA monomer is present in the brain. If an aS aggregate appears in the brain under such circumstances, both the endogenous monomer and the KA monomer should be aggregated in a seed-dependent manner (Figures 17-i and ii). The resulting endogenous monomer-derived aggregate (WT + FWT) is considered to function as a seed itself and to promote the aggregation of the endogenous monomer and the KA monomer (Figures 17-A and B). On the other hand, since the KA monomer-derived aggregate (KA + FWT: Figure 17-ii) has significantly low seed activity, as shown in Figure 16, the KA monomer-derived aggregate is unlikely to promote aggregation of endogenous monomers.

In other words, since it can be said that the KA monomer-derived aggregate has lower seed activity than an endogenous monomer-derived aggregate, it is considered that total generation of endogenous monomer-derived aggregates (i.e., aggregates with high pathogenicity) can be suppressed by allowing the KA monomers to express in the brain, or by delivering the KA monomers into the brain (Figures 17-B, C and D). Therefore, expression of the K43&45A or K43&45 delta monomer in the brain by an expression system using viruses such as adeno-associated virus, or by mRNA, plasmid, etc., or administration of the monomer itself may be useful as a novel treatment method for alpha-synucleinopathy.

### References

Fitzpatrick A.W.P et al. Cryo-EM structures of tau filaments from Alzheimer's disease. Nature 547: 185-190. 2017.
Goedert M. Cryo-EM structures of τ filaments from human brain. Essays in Biochemistry. 65: 949-959. 2021.
Masuda-Suzukake M. et al, Dextran sulphate-induced tau assemblies cause endogenous tau aggregation and propagation in wild-type mice. Brain Commun. 2: fcaa091. 2020.
Zhang W. et al, Heparin-induced tau filaments are polymorphic and differ from those in Alzheimer's and Pick's diseases. Elife. 8:e43584, 2019.

### [Example 2] Tau variants

### 1. Method

### Preparation of deletion mutation-type tau

Variant taus were prepared using KOD Plus Mutagenesis Kit (TOYOBO). Upon preparation of a ΔKP variant deleting Lys331 to Gln336 (amino acid residue numbers of tau 4R2N) and a ΔPG variant deleting Pro332 to Gly335 (amino acid residue numbers of tau 4R2N), RK172-human tau 4R1N (a vector for expression in *Escherichia coli*) or pcDNA3-human tau 4R1N (a vector for expression in cultured cells), which encodes wild-type tau (4R1N), was used as a template, and PCR was carried out using the following primers.
ΔKP-FW
   GTGGAAGTAAAATCTGAGAAGCTTG (SEQ ID No: 67)
ΔKP-RV
   ATGATGGATGTTGCCTAATGAGCCA (SEQ ID No: 68)
ΔPG-FW
   CAGGTGGAAGTAAAATCTGAGAAGC (SEQ ID No: 69)
ΔPG-RV
   TTTATGATGGATGTTGCCTAATGAG (SEQ ID No: 70)

The conditions for PCR are as follows.
1: 94°C, 2 minutes
2: 98°C, 10 seconds
3: 68°C, 7 minutes

The above cycles 2 and 3 are repeated 10 times.

To the amplified PCR product (50 µl), 1 µl of DpnI (TOYOBO) was added, and the obtained mixture was then incubated at 37°C for 1 hour to degrade the plasmid added as a template. An aliquot (2 µl) was taken from this reaction solution, and was then mixed with 7 µl of distilled water, 5 µl of Ligation-high and 1 µl of T4 polynucleotide kinase, and the thus obtained mixture was then incubated at 16°C for 1 hour. An aliquot (3 µl) was taken from this reaction solution, and was then mixed with 15 µl of *Escherichia coli* DH5 alpha competent cells, and then, the obtained mixture was incubated at 42°C for 45 seconds for transformation. To this reaction solution, 100 µl of LB medium was added, and the obtained mixture was then incubated at 37°C for 1 hour. Thereafter, the entire amount of the reaction mixture was seeded on a carbenicillin-resistant LB plate, and variant plasmids were then obtained from the obtained colonies. The introduced mutations were confirmed by performing DNA sequencing analysis on the obtained plasmids.

### Purification of recombinant tau monomers

The pRK172/human-4R1N tau plasmid (a wild type, or a ΔPG variant or a ΔKP variant) was transformed into *Escherichia coli* BL21 (DE3) for protein expression, was then seeded on an LB plate containing 50 µg/ml carbenicillin, and were then cultured at 37°C overnight. The cell mass was recovered and was added to 500 ml of 2 x YT medium supplemented with carbenicillin to a final concentration of 50 µg/ml, and was then cultured for 1 hour 15 minutes. Thereafter, IPTG was added to the cultured cells to a final concentration of 0.1 mM, and the cells were cultured again for 2 hours to induce expression. The culture medium was transferred into a centrifuge tube, and was then centrifuged at 4000 rpm at 4°C for 10 minutes. The cell mass was recovered, and was then cryopreserved at -80°C.

Thereafter, the cell mass was thawed, and was then suspended in 10 ml of tau purification buffer (50 mM Pipes-NaOH pH6.9, 1 mM ethylenediaminetetraacetic acid (EDTA), 1 mM dithiothreitol (DTT), and 0.5 mM phenylmethylsulfonyl fluoride (PMSF)). The suspension was transferred into a centrifuge tube, and was then subjected to ultrasonic disintegration on ice. The suspension was centrifuged at 15,000 rpm at 4°C for 15 minutes, and the supernatant was then recovered. To the obtained supernatant, 50 µl of 2-mercaptoethanol was added, and the obtained mixture was then heat-treated at 100°C for 5 minutes. After the heat treatment, the sample was centrifuged again at 15,000 rpm at 4°C for 15 minutes. The supernatant was recovered, and was then passed through an SP Sepharose^{™} Fast Flow (Cytiva) column (column volume: 3 ml) equilibrated with a tau purification buffer, so that the protein contained in the supernatant was adsorbed and was then washed with 30 ml of tau purification buffer.

Thereafter, the column was washed with 9 ml of tau purification buffer containing 0.1 M sodium chloride (NaCl), and the protein adsorbed on the column was then eluted with 9 ml of tau purification buffer containing 0.35 M NaCl. Ammonium sulfate was added to the eluate to 50% saturation, and the protein was then precipitated by leaving at rest overnight on ice. The obtained precipitate was centrifuged at 15,000 rpm at 4°C for 15 minutes, and the supernatant was removed. The thus obtained precipitate was dissolved in 1 ml of 30 mM Tris-HCl (pH 7.5), and was then desalted by dialysis in the same buffer as described above for 30 min (first time) and then overnight (second time). The obtained sample was centrifuged at 50,000 rpm at 4°C for 20 minutes, and the obtained supernatant was used as a recombinant tau monomer fraction. An aliquot of this fraction was analyzed by reversed-phase high-performance liquid chromatography (RT-HPLC), and the protein concentration was then calculated from the obtained peak area.

### Formation of tau fibers

To the purified recombinant tau monomer solution, 5 mM dithiothreitol (DTT) (left at rest for 10 minutes after addition), 0.2% sodium azide (NaN₃), and 200 µg/ml dextran sulfate were added in this order. The obtained mixture was shaken at 200 rpm at 37°C for 3 to 4 days, so as to aggregate tau monomers. This sample was centrifuged at 50,000 rpm at 25°C for 20 minutes, and tau fibers were recovered as precipitates. To the tau fibers, 500 µl of normal saline (Otsuka Pharmaceutical Co., Ltd.) was added, and the obtained mixture was then centrifuged under the same conditions as those described above to remove the remaining monomers. The tau fibers obtained as precipitates were resuspended in 150 µl of normal saline, and were then subjected to an ultrasonic treatment. An aliquot (10 µl) was taken from the resultant and was then mixed with 89 µl of 6 M guanidine hydrochloride and 1 µl of 0.1 M DTT, and the protein concentration in the tau fibers was measured by RT-HPLC.

### Measurement of fibrosis of tau monomers using thioflavin T (ThT)

A wild-type or variant tau monomer solution (3 mg/ml, 100 µl) was placed in a 96-well plate, and 10 mM DTT (left at rest for 10 minutes after addition), 50 mM Tris-HCl buffer (pH7.5), 0.2% NaN₃, 200 µg/ml dextran sulfate, and 40 µM thioflavin T (ThT) were added to the solution in this order, and were mixed. Each sample was shaken using a FLUOstar Omega plate reader (BMG LABTECH) at 37°C at 200 rpm, and the fluorescence intensity of ThT was measured over time at an excitation wavelength of 450 nm and at a fluorescence wavelength of 480 nm.

.

### Electron microscopic observation

2 µl of wild-type or variant tau fibers (0.2 mg/ml) was added to a collodion membrane-adhered mesh, and when the fibers were evenly distributed on the mesh, the sample was sucked out with a pipette and the remaining water was then wiped off with a Kim wipe. After that, 10 µl of 2% sodium phosphotungstate solution was added thereto, and was then left at rest at room temperature for several minutes. Thereafter, water was wiped off with a Kim wipe, and the mesh was left at rest at room temperature for several minutes, so that it was naturally dried. After drying, the mesh was subjected to JEM-1400 electron microscopy (JEOL) to observe the fiber structure of each aggregate.

### Measurement of seed activity of tau aggregate using ThT

A wild-type tau monomer solution (1 mg/ml, 100 µl) was placed in a 96-well plate, and 6.5 mM DTT (left at rest for 10 minutes after addition), 30 mM Tris-HCl buffer (pH 7.5), 0.2% NaN₃, 40 µM ThT were added to the solution in this order, and finally 2 µl of each aggregate (1 mg/ml) was added and mixed. The fluorescence intensity of ThT in each sample was measured over time, using an infinite M200 PRO/infinite M NANO + plate reader (TECAN), at 37°C, at an excitation wavelength of 442 nm and at a fluorescence wavelength of 485 nm.

### Seed activity of tau aggregate using cultured cells

The human neuroblastoma cell line SH-SY5Y was used as cultured cells. On a 6-well plate, 8 x 10⁵ cells per well were seeded, and were then cultured overnight. The next day, a wild-type tau expression plasmid (pcDNA3-human 4R1N tau) was introduced into the cells, using X-treamGENE9 (Roche). Specifically, Opti-MEM, the plasmid, and the X-treamGENE9 were gently mixed with one another at a ratio of 100 µl : 1 µg : 3 µl, and the obtained mixture was then left at rest at room temperature for 15 minutes. Thereafter, the mixed solution was added into the culture medium in each well. After about 3 hours had passed, 2 µl of recombinant tau aggregate was introduced into the cells. Specifically, a tau aggregate (1 mg/ml, 2 µl) was mixed with 60 µl of MultiFectam reagent (Promega), and the obtained mixture was then left at rest for 15 minutes. Thereafter, the total amount of the reaction mixture was added dropwise into each well. The treated cells were incubated in a CO₂ incubator for 3 days.

### Detection of insoluble tau by immunoblotting method

Cells, which had been treated with tau expression plasmids and aggregates, were recovered as follows, and were then prepared as a sample for immunoblotting. The culture medium in each well was removed with an aspirator, and 1 ml of sterilized phosphate buffered normal saline (PBS) was added thereto. The cells were peeled by pipetting and were recovered. The cells were recovered by performing centrifugation at 4500 rpm at 25°C for 5 minutes, and the recovered cells were then disrupted by adding thereto 300 µl of A68 buffer (10 mM Tris-HCl buffer (pH 7.5), 10% sucrose, 0.8 M NaCl, and 1 mM glycol ether diamine tetraacetic acid (EGTA)) containing 1% sarkosyl, and performing an ultrasonic treatment for about 1 minute.

Thereafter, 300 µl of A68 buffer containing 1% sarkosyl was further added, and the cells were then centrifuged at 50000 rpm at 25°C for 20 minutes. The obtained supernatant (300 µl) was recovered, and 75 µl of 5 x SDS buffer containing 5% 2-mercaptoethanol was added thereto, followed by performing a heat treatment at 100°C for 5 minutes. The resultant was used as a sarkosyl supernatant fraction (Sar-sup). On the other hand, the precipitate fraction was subjected to an ultrasonic treatment after addition of 55 µl of 2 x SDS buffer containing 5% 2-mercaptoethanol thereto, and it was then subjected to a heat treatment at 100°C for 5 minutes. The resultant was used as a sarkosyl-insoluble fraction (Sar-ppt).

These samples (Sar-sup and Sar-ppt) were electrophoresed on 7.5% polyacrylamide gel and were then transcribed onto poly(vinylidene fluoride) (PVDF) membrane (Millipore) under conditions of 200 mA and for 1 hour. The PVDF membrane was blocked with PBS containing 3% gelatin (Wako) for 10 minutes, and was then allowed to react overnight with a primary antibody diluted with PBS containing 10% bovine serum (T46: an antibody recognizing the C-terminus of tau: 2000-fold dilution: Cat# 13-6400, ThermoFisher; pS396: an antibody recognizing phosphorylation of the 396th residue Ser of tau: 2000-fold dilution: Cat# 44-752G, ThermoFisher; AT8: an antibody recognizing phosphorylation of the 202th residue Ser and the 205th residue Thr: 1000-fold dilution: Cat# MN1020, ThermoFisher).

Thereafter, the PVDF membrane was washed with Tris-buffered normal saline (TS), and was then allowed to react for 2 hours with a secondary antibody diluted with PBS containing 10% bovine serum (Biotin-Goat anti mouse IgG (Cat# BA-2000-1.5) or Biotin-Goat anti rabbit IgG ( Cat# BA-1000-1.5): 500-fold dilution, Vector), and thereafter, the membrane was washed with TS. Subsequently, the PVDF membrane was allowed to react with an avidin-biotin-labeled enzyme complex for 1 hour, was then washed with PBS, and was then treated with PBS containing 40 mg/ml 3,3'-diaminobenzidine (DAB: 100-fold dilution, Sigma-Aldrich), 80 mg/ml nickel chloride (50x dilution), 30% hydrogen peroxide solution (1000x ), 80 mg/ml nickel chloride (50-fold dilution), and 30% hydrogen peroxide water (1000-fold dilution), and the membrane protein bands were chromogenized. The coloration reaction was s terminated by washing with tap water.

### Results

### Purification of recombinant tau monomer

*Escherichia coli,* into which plasmids encoding wild type and variant tau had been introduced, were cultured in 500 mL of 2 x YT medium, and the cell mass was then recovered, and then, the cells were disrupted in a buffer solution. Since the tau protein is stable to heat, the cell mass lysate was heat-treated at 100°C for 5 minutes, so that other foreign proteins were denatured and were removed by centrifugation. The supernatant was passed through an SP-Sepharose column to adsorb tau, which was then eluted with a sodium chloride solution. After the eluate had been precipitated with ammonium sulfate, dialysis was performed to obtain a tau monomer. For the purpose of confirming the degree of purification, the samples at individual purification stages and the final product were analyzed by SDS-PAGE (Figure 18), and it was confirmed that the tau monomer with a molecular weight of interest was obtained.

### Aggregate formation of tau monomer

It is known that wild-type tau monomers are aggregated by shaking at 37°C in the presence of dextran sulfate [3]. It was investigated whether or not the two types of variant tau monomers prepared in this study are aggregated by shaking at 37°C in the presence of dextran sulfate, as with wild-type tau monomers. Wild-type and variant tau monomer (3 mg/ml), dextran sulfate, and ThT were added to a 96-well plate, and the fluorescence intensity of ThT was then measured over time at an excitation wavelength of 450 nm and a fluorescence wavelength of 480 nm, while shaking at 37°C. ThT does not bind to monomeric proteins, but binds specifically to protein aggregates having an amyloid fiber structure and emits fluorescence.

The fluorescence intensity of ThT was monitored over time. As a result, as shown in Figure 19, not only wild-type monomers but also ΔPG and ΔKP monomers showed an increase in the fluorescence intensity of ThT by shaking at 37°C in the presence of dextran sulfate, and it was revealed that these two types of variant tau monomers are also aggregated as well as the wild-type monomers. In addition, it was also suggested that aggregation of these variant monomers was slightly stronger than that of the wild-type.

### Electron microscopic observation of tau aggregates

The aggregates of wild-type and variant tau (0.2 mg/ml, 2 µl) were added onto a mesh, and were negatively stained with 2% phosphotungstic acid. These samples were observed by electron microscopy, and as a result, fibrous structures with a thickness of about 10 to 20 nm were observed in both of the wild-type and variant tau aggregates, as shown in Figure 20. Combined with the results of the section 4.2 above, it became clear that the ΔPG and ΔKP variant tau prepared in this study form an aggregate consisting of a fibrous structure in the presence of dextran sulfate, as with the wild-type tau.

### Seed activity of tau aggregate

If wild-type tau monomers are left at rest at 37°C without shaking, aggregates are hardly formed. However, it is known that when a small amount of wild-type aggregate is added to the monomer solution left at rest, the added aggregate functions as a seed and the tau monomers are aggregated. Thus, it was investigated whether or not the two types of ΔPG and ΔKP tau aggregates prepared in this study have the same seed activity in *vitro* as the wild-type aggregate. The wild-type tau monomer (1 mg/ml, 100 µl), each aggregate (1 mg/ml, 2 µl), and ThT were mixed with one another, and the obtained mixture was then incubated at 37°C.

The fluorescence intensity of ThT in these samples was measured over time at an excitation wavelength of 442 nm and at a fluorescence wavelength of 485 nm. The results are shown in Figure 21. When the wild-type tau monomer (+none) without addition of the aggregate was incubated at 37°C, the fluorescence intensity of ThT was not increased. However, when the wild-type aggregate was added to the wild-type tau monomer (+ WT), the fluorescence intensity of ThT was increased, and thus, it was confirmed that the wild-type aggregate has seed activity against the wild-type tau monomer.

On the other hand, when the two types of variant aggregates were added (+ΔPG and +ΔKP), the fluorescence intensity of ThT was lower than that in the case of addition of the wild-type aggregate. From these results, it was found that the seed activity of these two variant aggregates is lower than that of the wild-type aggregate. It became clear that, among others, the seeding effects of the ΔKP aggregate are much lower than those of the ΔPG aggregate.

### 4. Consideration

From the aforementioned results, it became clear that the variant tau monomers (ΔPG and ΔKP) are aggregated by shaking at 37°C in the presence of dextran sulfate, as with the wild-type tau monomers, and the variant tau monomers have a fibrous structure. Moreover, it was reconfirmed that the wild-type aggregate functions as a seed for the wild-type tau monomers and induces the accumulation thereof. On the other hand, the seed activity of both ΔPG and ΔKP variant aggregates was lower than that of the wild-type aggregate. From these results, it was demonstrated that the 331-336 residue region of tau, which has been focused in the present invention, does not affect so much its own aggregation and/or fibrillation in the presence of dextran sulfate, but plays an important role in seed activity.

### < References >

1. Schweighauser M. et al: Structures of α-synuclein filaments from multiple system atrophy. Nature, 585: 464-469 (2020).
2. Guerrero-Ferreira R. et al: New insights on the structure of alpha-synuclein fibrils using cryo-electron microscopy. Curr. Opin. Neurobiol., 61: 89-95 (2020).
3. Nonaka T. et al: Seeded aggregation and toxicity of α-synuclein and tau: cellular models of neurodegenerative diseases. J. Biol. Chem. 285(45): 34885-98 (2010).
4. Fujiwara H. et al: α-Synuclein is phosphorylated in synucleinopathy lesions. Nat Cell Biol 4:160-64 (2002).
5. Hasegawa M. et al: Phosphorylated α-synuclein is ubiquitinated in α-synucleinopathy lesions. J Biol Chem 277:49071-76 (2002).
6. Masuda-Suzukake M. et al: Prion-like spreading of pathological α-synuclein in brain. Brain, 136(Pt 4): 1128-1138 (2013).

### Sequence Listing Free Text

SEQ ID No: 17: n represents a, c, g, or t (existence positions: 127 to 129, 133 to 135, and 148 to 150)
SEQ ID No: 18: Xaa represents any given amino acid or deletion (existence positions: 43, 45, and 50)
SEQ ID No: 19: n represents a, c, g or t, or deletion (existence positions: 718 to 741)
SEQ ID No: 20: Xaa represents any given amino acid (existence positions: 240 and 241)
SEQ ID No: 20: Xaa represents Lys or deletion (existence position: 242)
SEQ ID No: 20: Xaa represents Pro or deletion (existence position: 243)
SEQ ID No: 20: Xaa represents Gly or deletion (existence position: 244)
SEQ ID No: 20: Xaa represents Gly or deletion (existence position: 245)
SEQ ID No: 20: Xaa represents Gly or deletion (existence position: 246)
SEQ ID No: 20: Xaa represents Gln or deletion (existence position: 247)
SEQ ID No: 21: n represents a, c, g or t, or deletion (existence positions: 805 to 828)
SEQ ID No: 22: Xaa represents any given amino acid (existence positions: 269 and 270)
SEQ ID No: 22: Xaa represents Lys or deletion (existence position: 271)
SEQ ID No: 22: Xaa represents Pro or deletion (existence position: 272)
SEQ ID No: 22: Xaa represents Gly or deletion (existence position: 273)
SEQ ID No: 22: Xaa represents Gly or deletion (existence position: 274)
SEQ ID No: 22: Xaa represents Gly or deletion (existence position: 275)
SEQ ID No: 22: Xaa represents Gln or deletion (existence position: 276)
SEQ ID No: 23: n represents a, c, g or t, or deletion (existence positions: 892 to 915)
SEQ ID No: 24: Xaa represents any given amino acid (existence positions: 298 and 299)
SEQ ID No: 24: Xaa represents Lys or deletion (existence position: 300)
SEQ ID No: 24: Xaa represents Pro or deletion (existence position: 301)
SEQ ID No: 24: Xaa represents Gly or deletion (existence position: 302)
SEQ ID No: 24: Xaa represents Gly or deletion (existence position: 303)
SEQ ID No: 24: Xaa represents Gly or deletion (existence position: 304)
SEQ ID No: 24: Xaa represents Gln or deletion (existence position: 305)
SEQ ID No: 25: n represents a, c, g or t, or deletion (existence positions: 811 to 834)
SEQ ID No: 26: Xaa represents any given amino acid (existence positions: 271 and 272)
SEQ ID No: 26: Xaa represents Lys or deletion (existence position: 273)
SEQ ID No: 26: Xaa represents Pro or deletion (existence position: 274)
SEQ ID No: 26: Xaa represents Gly or deletion (existence position: 275)
SEQ ID No: 26: Xaa represents Gly or deletion (existence position: 276)
SEQ ID No: 26: Xaa represents Gly or deletion (existence position: 277)
SEQ ID No: 26: Xaa represents Gln or deletion (existence position: 278)
SEQ ID No: 27: n represents a, c, g or t, or deletion (existence positions: 898 to 921)
SEQ ID No: 28: Xaa represents any given amino acid (existence positions: 300 and 301)
SEQ ID No: 28: Xaa represents Lys or deletion (existence position: 302)
SEQ ID No: 28: Xaa represents Pro or deletion (existence position: 303)
SEQ ID No: 28: Xaa represents Gly or deletion (existence position: 304)
SEQ ID No: 28: Xaa represents Gly or deletion (existence position: 305)
SEQ ID No: 28: Xaa represents Gly or deletion (existence position: 306)
SEQ ID No: 28: Xaa represents Gln or deletion (existence position: 307)
SEQ ID No: 29: n represents a, c, g or t, or deletion (existence positions: 985 to 1008)
SEQ ID No: 30: Xaa represents any given amino acid (existence positions: 329 and 330)
SEQ ID No: 30: Xaa represents Lys or deletion (existence position: 331)
SEQ ID No: 30: Xaa represents Pro or deletion (existence position: 332)
SEQ ID No: 30: Xaa represents Gly or deletion (existence position: 333)
SEQ ID No: 30: Xaa represents Gly or deletion (existence position: 334)
SEQ ID No: 30: Xaa represents Gly or deletion (existence position: 335)
SEQ ID No: 30: Xaa represents Gln or deletion (existence position: 336)
SEQ ID No: 31: n represents a, c, g or t, or deletion (existence positions: 82 to 84, 100 to 102, and 106 to 108)
SEQ ID No: 32: Xaa represents any given amino acid or deletion (existence positions: 28, 34, and 36)
SEQ ID No: 33: n represents a, c, g, or t (existence positions: 127 to 129, 133 to 135, and 148 to 150)
SEQ ID No: 34: Xaa represents any given amino acid (existence positions: 43, 45, and 50)
SEQ ID No: 35: n represents a, c, g, or t (existence positions: 718 to 726)
SEQ ID No: 36: Xaa represents any given amino acid (existence positions: 240 to 242)
SEQ ID No: 37: n represents a, c, g, or t (existence positions: 805 to 813)
SEQ ID No: 38: Xaa represents any given amino acid (existence positions: 269 to 271)
SEQ ID No: 39: n represents a, c, g, or t (existence positions: 892 to 900)
SEQ ID No: 40: Xaa represents any given amino acid (existence positions: 298 to 300)
SEQ ID No: 41: n represents a, c, g, or t (existence positions: 811 to 819)
SEQ ID No: 42: Xaa represents any given amino acid (existence positions: 271 to 273)
SEQ ID No: 43: n represents a, c, g, or t (existence positions: 898 to 906)
SEQ ID No: 44: Xaa represents any given amino acid (existence positions: 300 to 302)
SEQ ID No: 45: n represents a, c, g, or t (existence positions: 985 to 993)
SEQ ID No: 46: Xaa represents any given amino acid (existence positions: 329 to 331)
SEQ ID No: 47: n represents a, c, g or t, or deletion (existence positions: 82 to 84, 100 to 102, and 106 to 108)
SEQ ID No: 48: Xaa represents any given amino acid or deletion (existence positions: 28, 34, and 36)
SEQ ID No: 49 to 70: Synthetic DNAs

## Claims

1. A variant neurodegenerative disease-associated protein, which comprises an amino acid sequence comprising a deletion, substitution or addition of one or several amino acids in the amino acid sequence of the interaction region of two molecules of protofilaments (PF) in the amino acid sequence of a neurodegenerative disease-associated protein, and in which seed activity that functions as a nucleus of an aggregate of the neurodegenerative disease-associated protein is reduced to 70% or less of the seed activity of a wild-type neurodegenerative disease-associated protein.

2. The variant neurodegenerative disease-associated protein according to claim 1, wherein the neurodegenerative disease-associated protein is any protein selected from alpha-synuclein, tau and amyloid beta.

3. The variant neurodegenerative disease-associated protein according to claim 2, wherein the neurodegenerative disease-associated protein is alpha-synuclein, and one or several basic amino acids are deleted or substituted.

4. The variant neurodegenerative disease-associated protein according to claim 3, wherein the basic amino acid is at least one selected from Lys43, Lys45 and His50.

5. The variant neurodegenerative disease-associated protein according to claim 3, wherein the amino acid sequence of the alpha-synuclein is as set forth in SEQ ID No: 2.

6. The variant neurodegenerative disease-associated protein according to claim 2, wherein the neurodegenerative disease-associated protein is tau, and one or several basic amino acids or an amino acid sequence comprising an amino acid sequence shown as PGGG are deleted or substituted.

7. The variant neurodegenerative disease-associated protein according to claim 6, wherein the amino acid sequence comprising the amino acid sequence shown as PGGG is shown as KPGGGQ.

8. The variant neurodegenerative disease-associated protein according to claim 6, wherein the basic amino acid is a basic amino acid comprised in any isoform selected from the 3R0N, 3R1N, 3R2N, 4R0N, 4R1N and 4R2N isoforms of tau, and it is at least one of His329, His330 and Lys331 in the 4R2N isoform, or at least one selected from basic amino acids corresponding to the His329, His330 and Lys331 in the isoforms other than the 4R2N.

9. The variant neurodegenerative disease-associated protein according to claim 6, wherein the amino acid sequence of the tau is as set forth in SEQ ID No: 4, 6, 8, 10, 12 or 14.

10. The variant neurodegenerative disease-associated protein according to claim 2, wherein the neurodegenerative disease-associated protein is amyloid beta, and one or several hydrophobic amino acids or basic amino acids are deleted or substituted.

11. The variant neurodegenerative disease-associated protein according to claim 10, wherein the hydrophobic amino acid or the basic amino acid is at least one selected from Leu34, Val36 and Lys28.

12. The variant neurodegenerative disease-associated protein according to claim 10, wherein the amino acid sequence of the amyloid beta is as set forth in SEQ ID No: 16.

13. A nucleic acid encoding the variant neurodegenerative disease-associated protein according to any one of claims 1 to 12.

14. A recombinant vector comprising the nucleic acid according to claim 13.

15. An aggregate of the neurodegenerative disease-associated protein, in which the variant neurodegenerative disease-associated protein according to any one of claims 1 to 12 is aggregated.

16. An antibody against the aggregate according to claim 15.

17. A pharmaceutical composition against neurodegenerative disease, comprising the variant neurodegenerative disease-associated protein according to any one of claims 1 to 12.

18. A pharmaceutical composition against neurodegenerative disease, comprising the nucleic acid according to claim 13.

19. A pharmaceutical composition against neurodegenerative disease, comprising the recombinant vector according to claim 14.

20. A vaccine against neurodegenerative disease, comprising the aggregate according to claim 15.

21. A variant neurodegenerative disease-associated protein: which comprises an amino acid sequence comprising a substitution of one or several hydrophobic amino acids or basic amino acids with basic amino acids in the amino acid sequence of the interaction region (PF interaction region) of two molecules of protofilaments (PF) in the amino acid sequence of a neurodegenerative disease-associated protein (provided that when a basic amino acid(s) are substituted, they are substituted with other basic amino acid(s) having a higher charge than the concerned basic amino acids), or an amino acid sequence comprising an addition of one or several basic amino acids to the PF interaction region; and which has seed activity of functioning as a nucleus of an aggregate of the neurodegenerative disease-associated protein that is 71% or more compared with the seed activity of a wild-type neurodegenerative disease-associated protein.

22. The variant neurodegenerative disease-associated protein according to claim 21, wherein the neurodegenerative disease-associated protein is any protein selected from alpha-synuclein, tau and amyloid beta.

23. The variant neurodegenerative disease-associated protein according to claim 22, wherein the neurodegenerative disease-associated protein is alpha-synuclein.

24. The variant neurodegenerative disease-associated protein according to claim 23, wherein the one or several basic amino acids in the PF interaction region are at least one selected from Lys43, Lys45 and His50.

25. The variant neurodegenerative disease-associated protein according to claim 23, wherein the amino acid sequence of the alpha-synuclein is as set forth in SEQ ID No: 2.

26. The variant neurodegenerative disease-associated protein according to claim 22, wherein the neurodegenerative disease-associated protein is tau.

27. The variant neurodegenerative disease-associated protein according to claim 26, wherein the one or several basic amino acids in the PF interaction region are basic amino acids comprised in any isoform selected from the 3R0N, 3R1N, 3R2N, 4R0N, 4R1N and 4R2N isoforms of tau, and the basic amino acid(s) are at least one of His329, His330 and Lys331 in the 4R2N isoform, or at least one selected from basic amino acids corresponding to the His329, His330 and Lys331 in the isoforms other than the 4R2N.

28. The variant neurodegenerative disease-associated protein according to claim 26, wherein the amino acid sequence of the tau is as set forth in SEQ ID No: 4, 6, 8, 10, 12 or 14.

29. The variant neurodegenerative disease-associated protein according to claim 22, wherein the neurodegenerative disease-associated protein is amyloid beta.

30. The variant neurodegenerative disease-associated protein according to claim 29, wherein the one or several hydrophobic amino acids or basic amino acids in the PF interaction region are at least one selected from Leu34, Val36 and Lys28.

31. The variant neurodegenerative disease-associated protein according to claim 29, wherein the amino acid sequence of the amyloid beta is as set forth in SEQ ID No: 16.

32. An aggregate of the neurodegenerative disease-associated protein, in which the variant neurodegenerative disease-associated protein according to any one of claims 24 to 34 is aggregated.

33. A cell or a non-human animal, into which the aggregate according to claim 32 is introduced.

34. A cell model or a non-human animal model of variant neurodegenerative disease, comprising the cell or non-human animal according to claim 33.

35. A nucleic acid encoding the variant neurodegenerative disease-associated protein according to any one of claims 21 to 31.

36. A recombinant vector comprising the nucleic acid according to claim 35.

37. A transformed cell or a transformed non-human animal, comprising the recombinant vector according to claim 36.

38. A cell model or a non-human animal model of neurodegenerative disease, comprising the transformed cell or the transformed non-human animal according to claim 37.

39. A method of screening for a therapeutic agent for neurodegenerative disease, which is **characterized in that** it comprises contacting or administering a candidate substance to be tested to the cell model or the non-human animal model according to claim 34 or the cell model or the non-human animal model according to claim 38.

40. A kit of screening for a therapeutic agent for neurodegenerative disease, comprising at least one selected from the group consisting of the aggregate according to claim 32, the nucleic acid according to claim 35, the recombinant vector according to claim 36, the transformed cell or the transformed non-human animal according to claim 37, and the transformed cell or the transformed non-human animal according to claim 38.
